# EUROPEAN PATENT APPLICATION

(11) **EP 4 814 124 A1**
(43) Date of publication of application: **30.09.2026**
(21) Application number: 24894138.7
(22) Date of filing: 19.11.2024
(51) Int. Cl.: C12N 15/861, A61K 35/761, A61K 38/17, A61K 48/00, A61P 15/00, A61P 35/00, C12N 15/12

(54) **GENE THERAPEUTIC AGENT FOR CANCER**

(30) Priority: 20.11.2023 JP 2023196621
(71) Applicant: National University Corporation Kobe University, Kobe-shi, Hyogo 657-8501 (JP)
(72) Inventor: SHIRAKAWA, Toshiro, Kobe-shi, Hyogo 657-8501 (JP); A, Ruhan, Kobe-shi, Hyogo 657-8501 (JP); KITAGAWA, Koichi, Kobe-shi, Hyogo 657-8501 (JP)
(74) Representative: Regimbeau
(86) International application number: PCT/JP2024/040927
(87) International publication number: WO 2025/110147

(57) **Abstract**

The present disclosure provides a cancer gene therapy technique having an enhanced cancer suppressive effect. The present inventors found that an excellent cancer suppressive effect is attainable by an oncolytic adenoviral vector comprising a nucleic acid that comprises an artificial gene in which a CD44 extracellular part, a Notch core region, and HIF-3α4 are fused, and E1A gene and E1B gene placed under the control of the COX-2 promoter, wherein the artificial gene, E1A gene, and E1B gene are arranged in the same orientation. The present disclosure was thus completed.

## Description

### Technical Field

The present disclosure relates to, for example, a novel gene therapeutic agent for cancer or a novel cancer gene therapy method, and an active ingredient thereof. The contents of all of the documents described in the present specification (including NPL 1 to NPL 12 cited below as prior art documents) are incorporated herein by reference.

### Background Art

In recent years, gene therapy using viral vectors has attracted attention as a new therapy method for cancer. This method makes use of the characteristics of various viruses and uses viruses as gene "carriers," i.e., viral vectors. One gene transfer method is in vivo gene therapy, in which a recombinant viral vector incorporated with a therapeutic gene is administered directly into the body to treat the disease. Cancer gene therapy differs from existing therapies, such as chemotherapy, in terms of its mechanism of action. In cancer gene therapy, genes are introduced into cancer cells to directly inhibit genes involved in cancer development and proliferation, or cancer-suppressor genes are introduced into cells to directly induce cell death. Therefore, cancer gene therapy is expected to be effective for cancers that are difficult to treat with existing chemotherapy and radiotherapy.

### Citation List

### Patent Literature

PTL 1: WO2021/131944

### Non-patent Literature

NPL 1: Kosaki R., Watanabe K., Yamaguchi Y. (1999). Overproduction of hyaluronan by expression of the hyaluronan synthase Has2 enhances anchorage-independent growth and tumorigenicity. Cancer Res. 1999 Mar 1; 59(5): 1141-5.
NPL 2: Bourguignon L. Y., Zhu H., Shao L., Chen Y. W. (2001). CD44 interaction with c-Src kinase promotes cortactin-mediated cytoskeleton function and hyaluronic acid-dependent ovarian tumor cell migration. J Biol Chem. 2001 Mar 9; 276(10): 7327-36. Epub 2000 Nov 17.
NPL 3: Fujita Y., Kitagawa M., Nakamura S., Azuma K., Ishii G., Higashi M., Kishi H., Hiwasa T., Koda K., Nakajima N., Harigaya K. (2002). CD44 signaling through focal adhesion kinase and its anti-apoptotic effect. FEBS Lett. 2002 Sep 25; 528(1-3): 101-8.
NPL 4: Bazil V., Horejsi V. (1992). Shedding of the CD44 adhesion molecule from leukocytes induced by anti-CD44 monoclonal antibody simulating the effect of a natural receptor ligand. J Immunol. 1992 Aug 1; 149(3): 747-53.
NPL 5: Murakami D., Okamoto I., Nagano O., Kawano Y., Tomita T., Iwatsubo T., De Strooper B., Yumoto E., Saya H. (2003). Presenilin-dependent gamma-secretase activity mediates the intramembranous cleavage of CD44. Oncogene. 2003 Mar 13; 22(10): 1511-6.
NPL 6: Ueda M., Saji H. (2014). Radiolabeled Probes Targeting Hypoxia-Inducible Factor-1-Active Tumor Microenvironments. Scientific World Journal. 2014; 2014:165461. doi: 10.1155/2014/165461. Epub 2014 Aug 18.
NPL 7: Gao N., Shen L., Zhang Z., Leonard S. S., He H., Zhang X. G., Shi X., Jiang B. H. (2004). Arsenite induces HIF-1alpha and VEGF through PI3K, Akt and reactive oxygen species in DU145 human prostate carcinoma cells. Mol Cell Biochem. 2004 Jan; 255(1-2):33-45.
NPL 8: Kopan R., Ilagan M. X. (2009). The canonical Notch signaling pathway: unfolding the activation mechanism. Cell. 2009 Apr 17; 137(2): 216-33. doi: 10.1016/j.cell.2009.03.045.
NPL 9: Shona, T., Graeme, J. (2009). A Cancer Gene Therapy Approach that Targets Tumor-associated Hyaluronan. Cancer Growth and Metastasis 2009 (2) November 2009 with 31 Reads.
NPL 10: Morsut L., Roybal K. T., Xiong X., Gordley R. M., Coyle S. M., Thomson M., Lim W. A. (2016). Engineering Customized Cell Sensing and Response Behaviors Using Synthetic Notch Receptors. Cell. 2016 Feb 11; 164(4): 780-91. doi: 10.1016/j.cell.2016.01.012. Epub 2016 Jan 28.
NPL 11: Makino Y., Cao R., Svensson K., Bertilsson G., Asman M., Tanaka H., Cao Y., Berkenstam A., Poellinger L. (2001). Inhibitory PAS domain protein is a negative regulator of hypoxia-inducible gene expression. Nature. 2001 Nov 29; 414(6863): 550-4.
NPL 12: Maynard M. A., Evans A. J., Hosomi T., Hara S., Jewett M. A., Ohh M. (2005). Human HIF-3alpha4 is a dominant-negative regulator of HIF-1 and is down-regulated in renal cell carcinoma. FASEB J. 2005 Sep; 19(11): 1396-406. (2)

### Summary of Invention

### Technical Problem

The present inventors have found so far that an artificial gene in which CD44 (in particular, the extracellular part), Notch (in particular, the core region), and HIF-3α4 are fused can exhibit a cancer suppressive effect (PTL 1). The present inventors have made further improvements and have primarily aimed to provide a cancer gene therapy technique having an enhanced cancer suppressive effect.

### Solution to Problem

The present inventors found that it might be possible to achieve the above aim by an oncolytic adenoviral vector comprising a nucleic acid that comprises an artificial gene in which a CD44 extracellular part, a Notch core region, and HIF-3α4 are fused, and E1A gene and E1B gene placed under the control of the COX-2 promoter, wherein the artificial gene, E1A gene, and E1B gene are arranged in the same orientation. The present inventors made further improvements, which led to the completion of the present disclosure.

The present disclosure includes, for example, the main subjects described in the following items.

### Item 1.

An oncolytic adenoviral vector comprising a nucleic acid, the nucleic acid comprising:
a base sequence (I) having a structure in which the following base sequences are linked in the order of (A)-(B)-(C):
   (A) a base sequence encoding a protein having a CD44 extracellular function,
   (B) a base sequence encoding a protein having a Notch core region function, and
   (C) a base sequence encoding a protein having an HIF-3α4 function; and
a base sequence (II) comprising:
   (D) a base sequence containing a COX-2 promoter;
   (E) a base sequence encoding E1A protein placed under the control of the COX-2 promoter; and
   (F) a base sequence encoding E1B protein placed under the control of the COX-2 promoter,
wherein the base sequence (I) and the base sequence (II) are arranged in the same orientation.

### Item 2.

An oncolytic adenoviral vector comprising a nucleic acid, the nucleic acid comprising:
a base sequence (I) having a structure in which a base sequence (A), a base sequence (B), and a base sequence (C) are linked in the order of (A)-(B)-(C); and
a base sequence (II) comprising:
   (D) a base sequence containing a COX-2 promoter;
   (E) a base sequence encoding E1A protein placed under the control of the COX-2 promoter; and
   (F) a base sequence encoding E1B protein placed under the control of the COX-2 promoter,

wherein the base sequence (I) and the base sequence (II) are arranged in the same orientation,
the base sequence (A) being:
   (a-1): a base sequence consisting of the base sequence of SEQ ID NO: 1, or
   (a-2): a base sequence consisting of a base sequence having a sequence identity of 85% or more to the base sequence of (a-1), and encoding a protein that can bind to hyaluronic acid, the base sequence (B) being:
      (b-1): a base sequence consisting of the base sequence of SEQ ID NO: 2, or
      (b-2): a base sequence consisting of a base sequence having a sequence identity of 85% or more to the base sequence of (b-1), and encoding a protein that can be cleaved by a protease, the base sequence (C) being:
         (c-1): a base sequence consisting of the base sequence of SEQ ID NO: 3, or
         (c-2): a base sequence consisting of a base sequence having a sequence identity of 85% or more to the base sequence of (c-1), and encoding a protein that can bind to HIF-1α,
the base sequence (D) being:
   (d-1): a base sequence consisting of the base sequence of SEQ ID NO: 4, or
   (d-2): a base sequence consisting of a base sequence having a sequence identity of 85% or more to the base sequence of (d-1), and encoding a promoter whose expression is induced under COX-2 expression-inducing conditions,
the base sequence (E) being:
   (e-1): a base sequence consisting of the base sequence of SEQ ID NO: 5, or
   (e-2): a base sequence consisting of a base sequence having a sequence identity of 85% or more to the base sequence of (e-1), and encoding a protein having E1A function, and
the base sequence (F) being:
   (f-1): a base sequence consisting of the base sequence of SEQ ID NO: 6, or
   (f-2): a base sequence consisting of a base sequence having a sequence identity of 85% or more to the base sequence of (f-1), and encoding a protein having E1B function.

### Item 3.

The oncolytic adenoviral vector according to Item 1, wherein
the base sequence (A) is:
   (a-1): a base sequence consisting of the base sequence of SEQ ID NO: 1, or
   (a-2): a base sequence consisting of a base sequence having a sequence identity of 85% or more to the base sequence of (a-1), and encoding a protein that can bind to hyaluronic acid;
the base sequence (B) is:
   (b-1): a base sequence consisting of the base sequence of SEQ ID NO: 2, or
   (b-2): a nucleic acid consisting of a base sequence having a sequence identity of 85% or more to the base sequence of (b-1), and encoding a protein that can be cleaved by a protease in a protein encoded by the nucleic acid having a structure in which (A), (B), and (C) are linked in this order,
the base sequence (C) is:
   (c-1): a nucleic acid consisting of the base sequence of SEQ ID NO: 3, or
   (c-2): a nucleic acid consisting of a base sequence having a sequence identity of 85% or more to the base sequence of (c-1), and encoding a protein that can bind to HIF-1α,
the base sequence (D) is:
   (d-1): a base sequence consisting of the base sequence of SEQ ID NO: 4, or
   (d-2): a base sequence consisting of a base sequence having a sequence identity of 85% or more to the base sequence of (d-1), and encoding a promoter whose expression is induced under COX-2 expression-inducing conditions,
the base sequence (E) is:
   (e-1): a base sequence consisting of the base sequence of SEQ ID NO: 5, or
   (e-2): a base sequence consisting of a base sequence having a sequence identity of 85% or more to the base sequence of (e-1), and encoding a protein having E1A function, and
the base sequence (F) is:
   (f-1): a base sequence consisting of the base sequence of SEQ ID NO: 6, or
   (f-2): a base sequence consisting of a base sequence having a sequence identity of 85% or more to the base sequence of (f-1), and encoding a protein having E1B function.

### Item 4.

An oncolytic adenoviral vector comprising a nucleic acid, the nucleic acid comprising:
a base sequence (III) being:
   (g-1) a base sequence consisting of the base sequence of SEQ ID NO: 7,
   (g-2) a base sequence consisting of a base sequence having a sequence identity of 85% or more to the base sequence of SEQ ID NO: 7, and having a structure in which a portion encoding a protein that can bind to hyaluronic acid, a portion encoding a protein that can be cleaved by a protease, and a portion encoding a protein that can bind to HIF-1α are linked in this order, or
   (g-3) a base sequence consisting of a base sequence having a sequence identity of 85% or more to the base sequence of SEQ ID NO: 7, and encoding a protein having an anticancer effect; and
a base sequence (II) comprising:
   (D) a base sequence containing a COX-2 promoter;
   (E) a base sequence encoding E1A protein placed under the control of the COX-2 promoter; and
   (F) a base sequence encoding E1B protein placed under the control of the COX-2 promoter,

wherein the base sequence (III) and the base sequence (II) are arranged in the same orientation,
the base sequence (D) being:
   (d-1): a base sequence consisting of the base sequence of SEQ ID NO: 4, or
   (d-2): a base sequence consisting of a base sequence having a sequence identity of 85% or more to the base sequence of (d-1), and encoding a promoter whose expression is induced under COX-2 expression-inducing conditions,
the base sequence (E) being:
   (e-1): a base sequence consisting of the base sequence of SEQ ID NO: 5, or
   (e-2): a base sequence consisting of a base sequence having a sequence identity of 85% or more to the base sequence of (e-1), and encoding a protein having E1A function, and
the base sequence (F) being:
   (f-1): a base sequence consisting of the base sequence of SEQ ID NO: 6, or
   (f-2): a base sequence consisting of a base sequence having a sequence identity of 85% or more to the base sequence of (f-1), and encoding a protein having E1B function.

### Item 5.

The oncolytic adenoviral vector according to any one of Items 1 to 4, further comprising (H) a base sequence encoding an Ad5/35 chimeric fiber protein.

### Item 6.

The oncolytic adenoviral vector according to Item 5, wherein the base sequence (H) is
(h-1): a base sequence consisting of the base sequence of SEQ ID NO: 8, or
(h-2): a base sequence consisting of a base sequence having a sequence identity of 85% or more to the base sequence of (h-1), and encoding a protein that can bind to CD46.

### Item 7.

The oncolytic adenoviral vector according to any one of Items 2 to 6, wherein the protease is an ADAM protease or γ-secretase.

### Item 8.

An anticancer composition comprising the oncolytic adenoviral vector according to any one of Items 1 to 7.

### Item 9.

The anticancer composition according to Item 8, which is an injection.

### Item 10.

The anticancer composition according to Item 8 or 9 for use in the treatment of COX-2 positive cancer.

### Item 11.

The anticancer composition according to any one of Items 8 to 10 for use in the treatment of at least one selected from the group consisting of bladder cancer, breast cancer, and prostate cancer.

### Item 12.

A method for treating cancer, comprising administering to a subject in need thereof a therapeutically effective dose of the oncolytic adenoviral vector according to any one of Items 1 to 7.

### Item 13.

The method according to Item 12, wherein the administration is by injection.

### Item 14.

The method according to Item 12 or 13, wherein the cancer is COX-2 positive cancer.

### Item 15.

The method according to any one of Items 12 to 14, wherein the cancer is at least one selected from the group consisting of bladder cancer, breast cancer, and prostate cancer.

### Item 16.

The oncolytic adenoviral vector according to any one of Items 1 to 7 for use in the treatment of cancer.

### Item 17.

The oncolytic adenoviral vector according to Item 16, wherein the oncolytic adenoviral vector is administered by injection.

### Item 18.

The oncolytic adenoviral vector according to Item 16 or 17, wherein the cancer is COX-2 positive cancer.

### Item 19.

The oncolytic adenoviral vector according to any one of Items 16 to 18, wherein the cancer is at least one selected from the group consisting of bladder cancer, breast cancer, and prostate cancer.

### Item 20.

An application of the oncolytic adenoviral vector according to any one of Items 1 to 7 for production of a cancer therapeutic agent.

### Item 21.

The application according to Item 20, wherein the therapeutic agent is an injection.

### Item 22.

The application according to Item 20 or 21, wherein the cancer is COX-2 positive cancer.

### Item 23.

The application according to any one of Items 20 to 22, wherein the cancer is at least one selected from the group consisting of bladder cancer, breast cancer, and prostate cancer.

### Advantageous Effects of Invention

The present disclosure can provide a novel gene therapeutic agent for cancer having a very high cancer suppressive effect.

### Brief Description of Drawings

Fig. 1 is a schematic diagram of CRAd-synNotch (forward) that is an adenoviral vector constructed in the Examples of the present disclosure.
Fig. 2 is an excerpt from the schematic diagram shown in Fig. 2, including CD44/Notch/HIF-3α4 fusion gene, Ad5/35 chimeric fiber gene, COX-2 promoter region, E1A gene, and E1B gene.
Fig. 3 is a schematic diagram of CRAd-GFP that is an adenoviral vector constructed in the Examples of the present disclosure.
Fig. 4 shows the results of expression analysis of CD46, CAR, and CD44 in BT474 cells in Test Example 2.1.1 (n = 3, average ± SE bars, ** p < 0.01).
Fig. 5 shows the results of expression analysis of CD46, CAR, and CD44 in T24 cells in Test Example 2.1.2 (n = 3, average ± SE bars, ** p < 0.01).
Fig. 6 shows the results of expression analysis of CD46, CAR, and CD44 in MDA-MB-231 cells in Test Example 2.1.3 (n = 3, average ± SE bars, ** p < 0.01).
Fig. 7 shows the results of expression analysis of CD46, CAR, and CD44 in DU145 cells in Test Example 2.1.4 (n = 3, average ± SE bars, ** p < 0.01).
Fig. 8 shows the results of expression quantification of COX-2 gene in T24 cells, MDA-MB-231 cells, and DU145 cells in Test Example 2.2 (n = 3, average ± SE bars, ** p < 0.01).
Fig. 9 shows the results of expression quantification of HAS-1, HAS-2, and HAS-3 genes in T24 cells in Test Example 2.3.1 (n = 3, average ± SE bars, ** p < 0.01, * p < 0.05).
Fig. 10 shows the results of expression quantification of HAS-1, HAS-2, and HAS-3 genes in MDA-MB-231 cells in Test Example 2.3.2 (n = 3, average ± SE bars, ** p < 0.01).
Fig. 11 shows the results of expression quantification of HAS-1, HAS-2, and HAS-3 genes in DU145 cells in Test Example 2.3.3 (n = 3, average ± SE bars, ** p < 0.01).
Fig. 12 shows the results of expression analysis of CD44 by Western blotting in Test Example 2.5.1.
Fig. 13 shows the results of expression quantification of HIF-3α4 gene by real-time RT-PCR in Test Example 2.5.2 (n = 3, average ± SE bars, ** p < 0.01).
Fig. 14 shows the results of expression analysis of CD44 by Western blotting in CRAd-synNotch (forward) clone 1 in Test Example 2.6.1.1.
Fig. 15 shows the results of expression analysis of CD44 by Western blotting in CRAd-synNotch (forward) clone 2 and clone 3 in Test Example 2.6.1.2.
Fig. 16 shows the results of expression quantification of CD44 gene and HIF-3α4 gene by real-time RT-PCR in Test Example 2.6.2 (n = 3, average ± SE bars, ** p < 0.01).
Fig. 17 shows the results of expression analysis of CD44 by Western blotting in MDA-MB-231 cells and T24 cells infected with CRAd-synNotch (forward) clone 3 after mass culture in Test Example 2.7.1.
Fig. 18 shows the results of expression quantification of CD44 gene and HIF-3α4 gene by real-time RT-PCR in T24 cells infected with CRAd-synNotch (forward) clone 3 after mass culture in Test Example 2.7.2 (n = 3, average ± SE bars, **p < 0.01).
Fig. 19 shows the results of evaluation of propagation ability of adenoviral vectors in Test Example 2.8 (n = 3, average ± SE bars, ** p < 0.01). The vertical axis represents the virus copy number (actual number). A: wtAd, B: ADX730, C: CRAd-GFP, and D: CRAd-synNotch.
Fig. 20 shows the results of evaluation of BT474 cell proliferation suppressive effect of CRAd-synNotch in Test Example 2.9.1 (n = 3, average ± SE bars). The vertical axis represents the absorbance measured at a wavelength of 492 nm.
Fig. 21 shows the results of evaluation of T24 cell proliferation suppressive effect of CRAd-synNotch in Test Example 2.9.2 (n = 3, average ± SE bars, ** p < 0.01). The vertical axis represents the absorbance measured at a wavelength of 492 nm.
Fig. 22 shows the results of evaluation of MDA-MB-231 cell proliferation suppressive effect of CRAd-synNotch in Test Example 2.9.3. The vertical axis represents the absorbance measured at a wavelength of 492 nm.
Fig. 23 shows the results of evaluation of DU145 cell proliferation suppressive effect of CRAd-synNotch in Test Example 2.9.4 (n = 3, average ± SE bars, ** p < 0.01, * p < 0.05). The vertical axis represents the absorbance measured at a wavelength of 492 nm.
Fig. 24 shows the results of evaluation of VGEF gene suppressive effect of CRAd-synNotch in Test Example 2.10.1 (n = 3, average ± SE bars, ** p < 0.01).
Fig. 25 shows the results of evaluation of SOX-2 gene suppressive effect of CRAd-synNotch in Test Example 2.10.2 (n = 3, average ± SE bars, ** p < 0.01).
Fig. 26 shows the results of evaluation of CCL2 gene suppressive effect of CRAd-synNotch in Test Example 2.10.3 (n = 3, average ± SE bars, ** p < 0.01).
Fig. 27 shows the results of evaluation of the bladder cancer treatment effect of CRAd-synNotch in vivo in Test Example 2.11. In Fig. 27, the vertical axis represents the tumor volume, and the horizontal axis represents the number of days from the start of administration of PBS, ADX730, CRAd-GFP, or CRAd-synNotch (n = 5, average ± SE bars, ** p < 0.01).
Fig. 28 shows the results of evaluation of the bladder cancer treatment effect of CRAd-synNotch in vivo in Test Example 2.11. In Fig. 28, the vertical axis represents the survival rate, and the horizontal axis represents the number of days from the start of administration of PBS, ADX730, CRAd-GFP, or CRAd-synNotch (n = 5).
Fig. 29 shows the base sequence of SEQ ID NO: 1.
Fig. 30 shows the base sequence of SEQ ID NO: 2.
Fig. 31 shows the base sequence of SEQ ID NO: 3.
Fig. 32 shows the base sequence of SEQ ID NO: 4.
Fig. 33 shows the base sequence of SEQ ID NO: 5.
Fig. 34 shows the base sequence of SEQ ID NO: 6.
Fig. 35 shows the base sequence of SEQ ID NO: 7.
Fig. 36 shows the base sequence of SEQ ID NO: 8.
Fig. 37 shows the base sequence of SEQ ID NO: 9.
Fig. 38 shows the amino acid sequences of SEQ ID NOs: 10 to 12.
Fig. 39 shows the amino acid sequence of SEQ ID NO: 13.
Fig. 40 shows the amino acid sequence of SEQ ID NO: 14.
Fig. 41 shows the amino acid sequence of SEQ ID NO: 15.
Fig. 42 shows the results of Western blotting using a CD44 antibody as a primary antibody (left) and the results of Western blotting using an HIF3α4 antibody as a primary antibody (right) after infecting T24 cells with CRAd-synNotch at an MOI of 50 and culturing them for 24 or 72 hours by the method described in item 1.7.2 in Test 2.12.
Fig. 43 shows the results of Western blotting using a CD44 antibody as a primary antibody (left) and the results of Western blotting using an HIF3α4 antibody as a primary antibody (right) after infecting DU145 cells with CRAd-synNotch at an MOI of 25 or 50 and culturing them for 24, 48, or 96 hours by the method described in item 1.7.2 in Test 2.12.
Fig. 44 shows the results of analysis of the expression levels of Cortactin, OCT4, and Nanog in T24 cells in Test 2.13 (n = 3, average ± SE bars, ** p < 0.01).
Fig. 45 shows the results of analysis of the expression levels of PHD3, GLUT1, and CyclinG2 in T24 cells in Test 2.13 (n = 3, average ± SE bars, ** p < 0.01, * p < 0.05).
Fig. 46 shows the results of analysis of the expression levels of Cortactin, OCT4, Nanog, and SOX-2 in DU145 cells in Test 2.13 (n = 3, average ± SE bars, ** p < 0.01).
Fig. 47 shows the results of analysis of the expression levels of VEGF, GLUT1, CyclinG2, and PHD3 in DU145 cells in Test 2.13 (n = 3, average ± SE bars, ** p < 0.01, * p < 0.05).
Fig. 48 shows the results of immunohistochemical staining of CD44 in a T24 mouse model in Test 2.14. Bar = 100 µm.

### Description of Embodiments

Embodiments included in the present disclosure are described in more detail below. The present disclosure preferably includes, for example, an oncolytic adenoviral vector incorporated with a specific nucleic acid, an anticancer composition containing the oncolytic adenoviral vector, and a method for treating cancer using the composition; however, it is not limited thereto. The present disclosure encompasses all that is disclosed herein and can be recognized by those skilled in the art.

The oncolytic adenoviral vector encompassed by the present disclosure is preferably an oncolytic adenoviral vector comprising a nucleic acid, the nucleic acid comprising:
a base sequence (I) having a structure in which the following base sequences are linked in the order of (A)-(B)-(C):
   (A) a base sequence encoding a protein having a CD44 extracellular function,
   (B) a base sequence encoding a protein having a Notch core region function, and
   (C) a base sequence encoding a protein having an HIF-3α4 function; and
a base sequence (II) comprising:
   (D) a base sequence containing a COX-2 promoter;
   (E) a base sequence encoding E1A protein placed under the control of the COX-2 promoter; and
   (F) a base sequence encoding E1B protein placed under the control of the COX-2 promoter,
wherein the base sequence (I) and the base sequence (II) are arranged in the same orientation.

The oncolytic adenoviral vector is sometimes referred to as "the oncolytic adenoviral vector of the present disclosure." The nucleic acid is sometimes referred to as "the nucleic acid of the present disclosure."

### 1. Oncolytic Adenoviral Vector

Oncolytic viruses are conditionally replicative viruses that can specifically propagate in target cancer cells. Oncolytic viruses generally cannot infect normal cells. Oncolytic viruses attach to specific receptors that are highly expressed on cancer cells and infect the cancer cells. Once taken up into the cancer cells, the oncolytic viruses self-replicate within the cells and produce viral proteins. In this process, the host cancer cells are lysed and killed. The oncolytic viruses that have propagated within the cells are then released from the dead cancer cells into the tumor microenvironment. Oncolytic adenovirus is a representative type of oncolytic virus.

The oncolytic adenoviral vector for use in the technique of the present disclosure is not particularly limited as long as the adenovirus derived from the vector is capable of infecting and propagating in cancer cells. However, the adenovirus is preferably an oncolytic adenoviral vector that infects cancer cells expressing a CD46 receptor and propagates in such cancer cells. Examples of such oncolytic adenoviral vectors include an oncolytic adenoviral vector having human adenovirus type 35 as a backbone. More specific examples include an oncolytic adenoviral vector derived from Ad1129-06 (OD 260 Inc.).

In a preferred embodiment of the oncolytic adenoviral vector of the present disclosure, the nucleic acid of the present disclosure is contained in an oncolytic adenoviral vector having human adenovirus type 35 as a backbone. In a more preferred embodiment of the oncolytic adenoviral vector of the present disclosure, the nucleic acid of the present disclosure is contained in an oncolytic adenoviral vector derived from pAd1129-06.

The oncolytic adenoviral vector of the present disclosure can be produced by known methods or methods that can be easily conceived from known methods, for example, genetic engineering techniques such as nucleic acid synthesis techniques, PCR, restriction enzyme digestion, DNA ligation techniques, and in vitro transcription techniques.

### 2. Nucleic Acid of the Present Disclosure

As described above, the nucleic acid of the present disclosure is an oncolytic adenoviral vector comprising a nucleic acid, the nucleic acid comprising:
a base sequence (I) having a structure in which the following base sequences are linked in the order of (A)-(B)-(C):
   (A) a base sequence encoding a protein having a CD44 extracellular function,
   (B) a base sequence encoding a protein having a Notch core region function, and
   (C) a base sequence encoding a protein having an HIF-3α4 function; and
a base sequence (II) comprising:
   (D) a base sequence containing a Cox-2 promoter;
   (E) a base sequence encoding E1A protein placed under the control of the Cox-2 promoter; and
   (F) a base sequence encoding E1B protein placed under the control of the Cox-2 promoter,
wherein the base sequence (I) and the base sequence (II) are arranged in the same orientation.

In the present disclosure, the term "nucleic acid" encompasses DNA, RNA, PNA, and the like. The nucleic acid in the present disclosure is preferably DNA or RNA, and particularly preferably DNA. The nucleic acid may also be subjected to known chemical modifications, as exemplified below. To prevent degradation by hydrolase such as nuclease, for example, the phosphate residue (phosphate) of each nucleotide can be substituted with a chemically modified phosphate residue, such as phosphorothioate (PS), methylphosphonate, or phosphorodithionate. The hydroxy group at position 2 of the sugar (ribose) of each ribonucleotide may be substituted with -OR (R represents, for example, CH₃ (2'-O-Me), CH₂CH₂OCH₃ (2'-O-MOE), CH₂CH₂NHC(NH)NH₂, CH₂CONHCH₃, CH₂CH₂CN, etc.). Further, the base moiety (pyrimidine, purine) may be subjected to chemical modification. Specifically, such chemical modification includes, for example, introduction of a methyl group or a cationic functional group into position 5 of the pyrimidine base, and substitution of the carbonyl group at position 2 of the pyrimidine base with thiocarbonyl. Non-limiting examples also include a nucleic acid in which phosphate moieties and/or hydroxyl moieties are modified, for example, with biotin, an amino group, a lower alkylamino group, and an acetyl group. A BNA (LNA) and the like can also preferably be used, in which the conformation of the sugar moiety is locked in the N form by bridging the 2' oxygen and the 4' carbon of the sugar moiety of the nucleotide.

### 2-1. Base Sequence (A)

The base sequence (A) is a base sequence encoding a protein having a CD44 extracellular function. CD44 is a receptor for hyaluronic acid and the like, and binds to a ligand (e.g., hyaluronic acid) to cluster and transmit signals. This is known to cause intracellular phenomena, including activation of various kinases involved in cell proliferation and cell migration, such as c-Src, FAK, and MAPK. It is also known that after such signal transmission, the intracellular domain translocates to the nucleus upon cleavage by a protease, and that the cleaved extracellular domain is released as soluble CD44. Due to such properties, CD44 is highly expressed in many types of cancer cells, such as colon cancer, breast cancer, stomach cancer, pancreatic cancer, and prostate cancer, and has been studied as a marker for cancer stem cells.

In the present disclosure, the CD44 extracellular function refers to a receptor function for ligands, and this function preferably includes, for example, hyaluronic acid-binding ability.

The protein having a CD44 extracellular function may be, for example, a protein of the entire extracellular part of CD44; or as long as the receptor function for ligands is not impaired, it may contain a cell membrane portion of CD44 or may be a protein comprising a portion of the extracellular part of CD44. Further, as long as this function is maintained, one or more amino acids may be deleted, substituted, or added in such a protein. The nucleic acid (A) is not particularly limited as long as it is a nucleic acid encoding such a polypeptide. Preferred examples of proteins having a CD44 extracellular function include a protein comprising the amino acid sequence of SEQ ID NO: 10. More preferred examples include a protein consisting of the amino acid sequence of SEQ ID NO: 10. The base sequence (A) is not particularly limited as long as it is a base sequence encoding such a polypeptide.

More specifically, examples of the base sequence (A) include the following (a-1) and (a-2).
(a-1): A base sequence consisting of the base sequence of SEQ ID NO: 1
(a-2): A base sequence consisting of a base sequence having a sequence identity of 85% or more to the base sequence of (a-1), and encoding a protein that can bind to hyaluronic acid

In the present disclosure, the term "identity" of base sequences refers to the degree of match between two or more comparable base sequences of two or more comparable nucleotide sequences. Thus, the higher the degree of match between two given base sequences, the higher the identity or similarity between those sequences. The level of sequence identity can be determined, for example, using FASTA, which is a sequence analysis tool, with default parameters, or using the BLAST algorithm developed by Karlin and Altschul (Karlin S., Altschul S. F., "Methods for assessing the statistical significance of molecular sequence features by using general scoring schemes" Proc Natl Acad Sci USA. 87: 2264-2268 (1990); and Karlin S., Altschul S. F., "Applications and statistics for multiple high-scoring segments in molecular sequences." Proc Natl Acad Sci USA, 90: 5873-7 (1993)). Specific techniques of these analysis methods are known, and the website of the National Center of Biotechnology Information (NCBI) (http://www.ncbi.nlm.nih.gov/) may be referred to. The term "identity" of amino acid sequences is also defined based on the above. The identity of amino acid sequences can be analyzed using a program called BLASTX.

A base sequence X' whose sequence identity to a base sequence X is not 100% may be, for example, a base sequence in which one or multiple bases are substituted, deleted, added, or inserted (preferably substituted) in the base sequence X. The term "multiple" refers to 2 to 100, preferably 2 to 80, more preferably 2 to 50, and even more preferably 2 to 10. The upper or lower limit of the range may be 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100.

In the present disclosure, when a base sequence X is a base sequence encoding a polypeptide, a base sequence X' whose sequence identity to the base sequence X is not 100% is preferably one that does not result in a frameshift relative to the base sequence X. When an amino acid sequence encoded by the base sequence X is referred to as "P" and an amino acid sequence encoded by the base sequence X' is referred to as "P'," it is particularly preferred that P' and P are identical to each other.

When the sequence identity of the amino acid sequence P' to the amino acid sequence P is not 100%, the sequence identity may be 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99% or more, preferably 80% or more, more preferably 85% or more, even more preferably 90% or more, and particularly preferably 95% or more. Examples of such P' include an amino acid sequence in which one or multiple amino acids are substituted, deleted, added, or inserted (preferably substituted, and more preferably conservatively substituted) in P. The term "multiple" refers to, for example, 2 to 20, preferably 2 to 10, more preferably 2 to 5, and even more preferably 2 or 3. The upper or lower limit of the range may be 2, 3, 4, 5, 6, 7, 8, 9, 10, 11, 12, 13, 14, 15, 16, 17, 18, 19, or 20.

The term "conservatively substituted" means that an amino acid residue is substituted with another amino acid residue having a similar side chain. For example, the substitution between amino acid residues having basic side chains, such as lysine, arginine, and histidine, is conservative substitution. In addition, the substitution between the following amino acid residues is also conservative substitution: amino acid residues having acidic side chains, such as aspartic acid and glutamic acid; amino acid residues having antistatic polar side chains, such as glycine, asparagine, glutamine, serine, threonine, tyrosine, and cysteine; amino acid residues having non-polar side chain, such as alanine, valine, leucine, isoleucine, proline, phenylalanine, methionine, and tryptophan; amino acid residues having β-branched side chains, such as threonine, valine, and isoleucine; and amino acid residues having aromatic side chains, such as tyrosine, phenylalanine, tryptophan, and histidine.

As described above, the base sequence (a-2) is a base sequence consisting of a base sequence having a sequence identity of 85% or more to the base sequence of (a-1), and encoding a protein that can bind to hyaluronic acid. The sequence identity may be 85, 86, 87, 88, 89, 90, 91, 92, 93, 94, 95, 96, 97, 98, or 99% or more, preferably 90% or more, more preferably 95% or more, and particularly preferably 99% or more.

In the present disclosure, that a protein is capable of binding to another protein means that the protein is capable of specifically interacting with the other protein. Whether a protein is capable of binding to another protein can be confirmed by methods known in the art. Examples of such methods include co-immunoprecipitation (Co-IP), pull-down assays, Western blotting, crosslinking methods, label transfer assays, interaction mapping, surface plasmon resonance (SPR), and fluorescence resonance energy transfer (FRET). Further, it is also possible to predict whether the protein is capable of binding to the other protein by computer simulation based on the amino acid sequences of the protein and the other protein.

### 2-2. Base Sequence (B)

The base sequence (B) is a base sequence encoding a protein having a Notch core region function. Notch is a receptor expressed on the surface of cells, and the Notch signaling system is one of the main signaling systems responsible for intercellular signaling. In many cases, the mechanism of intercellular signaling is such that the signaling cell produces and releases a soluble ligand, and the ligand binds to the receptor on the surface of a signal-receiving cell. Further, intracellular signaling pathways, including downstream phosphorylation cascades, are activated on the receiving cell side, and the activity of specific transcription factors changes, whereby gene expression is regulated. On the other hand, the Notch signaling system is characterized by signaling through direct interaction between adjacent cells. Notch, which acts as a receptor, binds to the ligand Delta or Serrate (Jagged in mammals) on the cell surface and further undergoes a conformational change due to the application of physical force, which causes the separation of the intracellular domain of Notch due to the action of a protein-cleaving enzyme (protease), such as ADAM protease or γ-secretase. The intracellular domain of Notch released from the cell membrane as described above translocates to the nucleus and regulates its transcription by interacting with transcription factors and coactivators upstream of the target gene.

In the present disclosure, the Notch core region is a region containing a site cleaved by the action of a protease, and the Notch core region function is a function of being cleavable (preferably being selectively separable) by a protease that can cleave the Notch core region.

The protein having a Notch core region function may be, for example, a protein containing only a portion of the Notch core region necessary for protease cleavage; or as long as it is cleaved by the action of a protease, it may be a protein containing, together with the portion necessary for protease cleavage, one or more (e.g., 1 to 30, 1 to 20, 1 to 10, or 1, 2, 3, 4, or 5) amino acids in front of and behind the portion necessary for protease cleavage. Further, as long as it is cleaved by the action of a protease, one or more (e.g., 1 to 30, 1 to 20, 1 to 10, or 1, 2, 3, 4, or 5) amino acids may be deleted, substituted, or added in such a protein. Preferred examples of proteins having a Notch core region function include a protein comprising the amino acid sequence of SEQ ID NO: 11. More preferred examples include a protein consisting of the amino acid sequence of SEQ ID NO: 11. The base sequence (B) is not limited as long as it is a base sequence encoding such a protein.

As described above, preferred examples of proteases include ADAM proteases and γ-secretase. A base sequence encoding a polypeptide that is cleaved by either or both of these proteases is more preferred. ADAM proteases are proteolytic enzymes belonging to the A Disintegrin and Metalloproteinase family.

Whether a protein can be cleaved by a protease can be confirmed by treating the protein with the protease, followed by electrophoresis (e.g., SDS-PAGE).

More specifically, examples of the base sequence (B) include the following (b-1) and (b-2).
(b-1): A base sequence consisting of the base sequence of SEQ ID NO: 2
(b-2): A base sequence consisting of a base sequence having a sequence identity of 85% or more to the base sequence of (b-1), and encoding a protein that can be cleaved by a protease
   (b-2) is more preferably (b-2'), which is a base sequence consisting of a base sequence having a sequence identity of 85% or more to the base sequence of (b-1), and encoding a protein encoded by a base sequence having a structure in which (A), (B), and (C) are linked in this order, the protein being cleavable by an intracellular protease when a ligand binds to a protein encoded by the base sequence (A).

The sequence identity may be 85, 86, 87, 88, 89, 90, 91, 92, 93, 94, 95, 96, 97, 98, or 99% or more, preferably 90% or more, more preferably 95% or more, and particularly preferably 99% or more.

### 2-3. Base Sequence (C)

The base sequence (C) is a base sequence encoding a protein having an HIF-3α4 function. Hypoxia inducible factor (HIF) is a transcription factor that is activated when intracellular hypoxia occurs, and is a heterodimer composed of HIF-1α and HIF-1β. HIF-1α has been shown to be inhibited not only by PHD-mediated degradation under normoxic conditions, but also by a transcription factor called inhibitory PAS domain protein (IPAS), which was found in mice. IPAS was identified as a splicing variant of HIF-3α, one of the HIFs. Although IPAS itself does not have transcriptional activity, its interaction with HIF-1α inhibits binding to DNA and suppresses the function of HIF-1α. In humans, HIF-3α4, identified as a splicing variant of HIF-3α, has been shown to function similarly to IPAS.

In the present disclosure, the HIF-3α4 function is a function that can inhibit HIF-1α. More specifically, it is a function that interacts with (binds to) HIF-1α.

The protein having an HIF-3α4 function may be, for example, HIF-3α4 itself, or as long as it can inhibit HIF-1α, may be a protein in which one or more (e.g., 1 to 30, 1 to 20, 1 to 10, or 1, 2, 3, 4, or 5) amino acids are further added to HIF-3α4. Further, as long as it can inhibit HIF-1α, one or more (e.g., 1 to 30, 1 to 20, 1 to 10, or 1, 2, 3, 4, or 5) amino acids may be deleted, substituted, or added in such a protein. For example, the protein having an HIF-3α4 function is preferably a protein comprising the amino acid sequence of SEQ ID NO: 12, and more preferably a protein consisting of the amino acid sequence of SEQ ID NO: 12. The base sequence (C) is not particularly limited as long as it is a base sequence encoding such a protein.

More specifically, examples of the base sequence (C) include the following (c-1) and (c-2).
(c-1): A base sequence consisting of the base sequence of SEQ ID NO: 3
(c-2): A base sequence consisting of a base sequence having a sequence identity of 85% or more to the base sequence of (c-1), and encoding a protein that can bind to HIF-1α

The sequence identity may be 85, 86, 87, 88, 89, 90, 91, 92, 93, 94, 95, 96, 97, 98, or 99% or more, preferably 90% or more, more preferably 95% or more, and particularly preferably 99% or more.

### 2-4. Base Sequence (I)

The base sequence (I) is a base sequence having a structure in which the base sequence (A), the base sequence (B), and the base sequence (C) are linked in the order of (A)-(B)-(C). In the base sequence (I), the base sequence (A) may be arranged on the 3' side or the 5' side, with the 5' side being preferred. The base sequence (A), the base sequence (B), and the base sequence (C) may be linked directly or linked via one or more linkers, with direct linkage being preferred. The linkers are not particularly limited as long as the effect of the present disclosure is not impaired. For example, a linker consisting of one or more bases may be used. When the base sequence (I) contains one or more linkers, the base length of each linker may be 1 to 1000 bp or 1 to 500 bp, preferably 1 to 100 bp or 1 to 50 bp, and particularly preferably 1 to 10 bp. The upper or lower limit of the range may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 350, 400, 450, 500, 550, 600, 650, 700, 750, 800, 850, 900, 950, or 1000 bp.

The base length of the base sequence (I) may be, for example, 8000 bp or less, preferably 7500, 7000, 6500, or 6000 bp or less, more preferably 5500, 5000, or 4500 bp or less, and particularly preferably 4000, 3500, or 3100 bp or less.

Examples of preferred embodiments of the base sequence (I) include the following (g-1), (g-2), and (g-3). These (g-1), (g-2), and (g-3) are sometimes collectively referred to as "the base sequence (III)."
(g-1) A base sequence consisting of the base sequence of SEQ ID NO: 7
(g-2) A base sequence consisting of base sequence having a sequence identity of 85% or more to the base sequence of SEQ ID NO: 7, and having a structure in which a portion encoding a protein that can bind to hyaluronic acid, a portion encoding a protein that can be cleaved by a protease, and a portion encoding a protein that can bind to HIF-1α are linked in this order
(g-3) A base sequence consisting of a base sequence having a sequence identity of 85% or more to the base sequence of SEQ ID NO: 7, and encoding a protein having an anticancer effect

The sequence identity may be 85, 86, 87, 88, 89, 90, 91, 92, 93, 94, 95, 96, 97, 98, or 99% or more, preferably 90% or more, more preferably 95% or more, and particularly preferably 99% or more.

Preferred examples of proteins encoded by the base sequence (I) include a protein comprising the amino acid sequence of SEQ ID NO: 13, and more preferred examples include a protein consisting of the amino acid sequence of SEQ ID NO: 13.

In the present disclosure, the term "anticancer effect" encompasses an effect of inhibiting the proliferation of cancer cells, an effect of killing cancer cells, an effect of reducing the number of cancer cells, and an effect of reducing tumor volume.

### 2-5. Base Sequence (D)

The base sequence (D) is a base sequence comprising a cyclooxygenase-2 (COX-2) promoter. COX-2 is involved in the conversion of arachidonic acid to prostaglandin H2. COX-2 is not expressed under normal conditions in most cells; however, its expression is known to be increased in an inflammatory state. In addition, it is known that the expression of COX-2 is increased in many cancer cells. In other words, a gene or protein encoded by a base sequence placed under the control of the COX-2 promoter may exhibit increased expression in cells in an inflammatory state or in cancer cells.

A promoter generally includes a transcription start site, an upstream sequence (5' side) thereof, and optionally a downstream sequence (3' side) thereof. Examples of COX-2 promoters include any DNA region containing the transcription start site in the DNA region between -10000 and +500, preferably -5000 and +200, and more preferably -2000 and +150, when the base of the transcription start site of the human or other COX-2 gene is defined as +1, the downstream (3' side) is defined as a positive value, and the upstream (5' side) is defined as 0 or a negative value. When multiple transcription start sites exist, the site with the highest transcription output can be selected. The base length of the base sequence (D) is, for example, 500 to 10000 bp, preferably 8000 to 5000 bp, and more preferably 1000 to 3000 bp.

The COX-2 promoter contained in the base sequence (D) may have base sequence mutations (e.g., substitution, deletion, insertion, addition, and the like) relative to the endogenous COX-2 promoter of an organism (preferably a human), as long as a gene or a protein under the control of the COX-2 promoter can exhibit increased expression in cells in an inflammatory state or in cancer cells. In this case, the COX-2 promoter contained in the base sequence (D) has an identity of, for example, 70% or more, preferably 80% or more, more preferably 90% or more, even more preferably 95% or more, still even more preferably 97% or more, and particularly preferably 99% or more, with the corresponding base sequence of the endogenous promoter. The mutation site is preferably other than, for example, known expression regulatory elements (e.g., inflammatory response regions, basic transcription factor binding regions, various activator binding regions, etc.).

More specifically, examples of the base sequence (D) include the following (d-1) and (d-2).
(d-1): A base sequence consisting of the base sequence of SEQ ID NO: 4
(d-2): A base sequence consisting of a base sequence having a sequence identity of 85% or more to the base sequence of (d-1), and encoding a promoter whose expression is induced under COX-2 expression-inducing conditions

The term "COX-2 expression-inducing conditions" refers to, for example, conditions in cells in an inflammatory state or in cancer cells. The phrase "expression is induced under COX-2 expression-inducing conditions" means that the gene or protein expression level under the control of the promoter is increased in cells in an inflammatory state or in cancer cells, as compared to the expression level in normal cells that are not in an inflammatory state.

In the present disclosure, the term "expression level" encompasses both the amount of transcription products and the amount of translation products. The expression level can be analyzed by conventionally known methods or methods that can be readily conceived from such known methods. Examples of such methods include reverse transcription quantitative PCR (RT-qPCR), RNA-seq, Western blotting, and immunohistochemical staining.

The sequence identity may be 85, 86, 87, 88, 89, 90, 91, 92, 93, 94, 95, 96, 97, 98, or 99% or more, preferably 90% or more, more preferably 95% or more, and particularly preferably 99% or more.

### 2-6. Base Sequence (E)

The base sequence (E) is a base sequence encoding E1A protein, and is placed under the control of the COX-2 promoter. Thus, the base sequence (E) is arranged on the 3' side of the base sequence (D). The base sequence (E) may be directly linked to the 3' end of the base sequence (D), or another base sequence (e.g., a linker, a base sequence (F), etc.) may be present between the base sequence (E) and the base sequence (D). In the latter case, the base length of the base sequence between the base sequence (E) and the base sequence (D) is not limited as long as the base sequence (E) is under the control of the COX-2 promoter.

E1A protein, together with E1B protein described below, is an essential factor for adenovirus propagation. More specifically, E1A protein is a non-structural protein and has a function of initiating virus replication upon transcription and translation during the initial stage of virus replication.

In the present disclosure, similarly to E1A protein, the term "E1A function" refers to a function of initiating virus replication upon transcription and translation during the initial stage of virus replication. The protein having E1A function may be E1A protein itself, or similarly to E1A protein, may be a protein in which one or more (e.g., 1 to 30; 1 to 20; 1 to 10; or 1, 2, 3, 4, or 5) amino acids are further added to E1A protein, as long as it can initiate virus replication upon transcription and translation during the initial stage of virus replication. Further, similarly to E1A protein, one or more (e.g., 1 to 30; 1 to 20; 1 to 10; or 1, 2, 3, 4, or 5) amino acids may be deleted, substituted, or added in such a protein, as long as it can initiate virus replication upon transcription and translation during the initial stage of virus replication. The base sequence (E) is not particularly limited as long as it is a base sequence encoding such a protein.

More specifically, examples of the base sequence (E) include the following (e-1) and (e-2).
(e-1): A base sequence consisting of the base sequence of SEQ ID NO: 5
(e-2): A base sequence consisting of a base sequence having a sequence identity of 85% or more to the base sequence of (e-1), and encoding a protein having E1A function

The sequence identity may be 85, 86, 87, 88, 89, 90, 91, 92, 93, 94, 95, 96, 97, 98, or 99% or more, preferably 80% or more, more preferably 85% or more, even more preferably 90% or more, and particularly preferably 95% or more.

### 2-7. Base Sequence (F)

The base sequence (F) is a base sequence encoding E1B protein, and is placed under the control of the COX-2 promoter. Thus, the base sequence (F) is arranged on the 3' side of base sequence (D). The base sequence (F) may be directly linked to the 3' end of the base sequence (D), or another base sequence (e.g., a linker, the base sequence (E), etc.) may be present between the base sequence (F) and the base sequence (D). In the latter case, the base length of the base sequence between the base sequence (F) and the base sequence (D) is not limited as long as the base sequence (F) is under the control of the COX-2 promoter.

E1B protein, together with E1A protein described above, is an essential factor for adenovirus propagation. More specifically, E1B protein is a protein essential for the formation of viral replication compartments, and controls replication and transcription of the viral genome, viral late mRNA biosynthesis, and like other functions.

In the present disclosure, similarly to E1B protein, the term "E1B function" refers to a function that controls replication and transcription of the viral genome, viral late mRNA biosynthesis, and like other functions, and is involved in the formation of viral replication compartments.

The protein having E1B function may be E1B protein itself, for example, or, similarly to E1B protein, may be a protein in which one or more (e.g., 1 to 30; 1 to 20; 1 to 10; or 1, 2, 3, 4, or 5) amino acids are further added to E1B protein, as long as it can control replication and transcription of the viral genome, viral late mRNA biosynthesis, and like other functions. Further, similarly to E1B protein, one or more (e.g., 1 to 30; 1 to 20; 1 to 10; or 1, 2, 3, 4, or 5) amino acids may be deleted, substituted, or added in such a protein, as long as it can control replication and transcription of the viral genome, viral late mRNA biosynthesis, and like other functions. The base sequence (F) is not particularly limited as long as it is a base sequence encoding such a protein.

More specifically, examples of the base sequence (F) include the following (f-1) and (f-2).
(f-1): A base sequence consisting of the base sequence of SEQ ID NO: 6
(f-2): A base sequence consisting of a base sequence having a sequence identity of 85% or more to the base sequence of (f-1), and encoding a protein having E1B function

The sequence identity may be 85, 86, 87, 88, 89, 90, 91, 92, 93, 94, 95, 96, 97, 98, or 99% or more, preferably 90% or more, more preferably 95% or more, and particularly preferably 99% or more.

### 2-8. Base Sequence (II)

The base sequence (II) is a base sequence comprising the base sequence (D), the base sequence (E), and the base sequence (F). As described above, the base sequence (E) and the base sequence (F) are placed under the control of the COX-2 promoter contained in the base sequence (D). Thus, the base sequence (D) is arranged upstream (5' side) of the base sequence (E) and the base sequence (F). Between the base sequence (E) and the base sequence (F), either one is arranged on the 5' side. In other words, the base sequence (II) may have a structure in which the base sequences are linked in the order of (D)-(E)-(F) from the 5' side or a structure in which the base sequences are linked in the order of (D)-(F)-(E) from the 5' side. The base sequence (D), the base sequence (E), and the base sequence (F) may be linked directly or linked via one or more linkers. The linkers are not particularly limited as long as the effect of the present disclosure is not impaired. For example, a linker consisting of one or more bases may be used. When the base sequence (II) contains one or more linkers, the base length of each linker may be 1 to 5000 bp, preferably 1 to 1000 bp, more preferably 1 to 500 bp, and particularly preferably 1 to 300 bp. The upper or lower limit of the range may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 350, 400, 450, 500, 550, 600, 650, 700, 750, 800, 850, 900, 950, 1000, 1500, 2000, 2500, 3000, 3500, 4000, 4500, or 5000 bp.

In the nucleic acid of the present disclosure, the base sequence (I) or the base sequence (III), and the base sequence (II) are arranged in the same orientation. In the present disclosure, that multiple base sequences are "arranged in the same orientation" means that transcription of each base sequence proceeds in the same direction. In the case of double-stranded nucleic acids, this can also be expressed as transcription being carried out using the same nucleic acid strand as a template (i.e., the sense strands are identical). Between the base sequence (I) or the base sequence (III) and the base sequence (II), either one may be arranged upstream.

### 2-9. Base Sequence (H)

Although it is not particularly limited, the nucleic acid of the present disclosure preferably further comprises, in addition to the base sequences (A) to (F), a base sequence (H), which is a base sequence encoding an Ad5/35 chimeric fiber protein. In the present disclosure, the term "Ad5/35 chimeric fiber protein" refers to a chimeric fiber protein comprising a portion or the entirety of a fiber protein derived from an adenovirus serotype 35 (Ad35) and a portion or the entirety of a fiber protein derived from an adenovirus serotype 5 (Ad5). In the present disclosure, an Ad5/35 chimeric fiber protein or a base sequence encoding an Ad5/35 chimeric fiber protein is sometimes simply described as "Ad5/35 fiber."

In order to introduce a nucleic acid into target cells using an Ad5 vector, which is widely used in gene therapy, it is necessary that the adenovirus receptor (CAR) be expressed in the target cells. Thus, in gene therapy using an Ad5 vector, a sufficient therapeutic effect cannot be expected in target cells in which CAR is not expressed or in which the expression level of CAR is low. Meanwhile, the Ad35 fiber protein is capable of binding to CD46, which is a membrane protein different from CAR. When the nucleic acid of the present disclosure comprises a base sequence encoding an Ad5/35 chimeric fiber protein, a nucleic acid can be introduced, via binding to CD46, into cells in which CAR is not expressed or in which the expression level of CAR is low. In other words, the nucleic acid of the present disclosure preferably comprises the base sequence (H), i.e., a base sequence encoding an Ad5/35 chimeric fiber protein, from the perspective of expecting a high anticancer effect regardless of the presence or absence of CAR expression and the level of CAR expression in the target cells.

As described above, in the present disclosure, the term "Ad5/35 chimeric fiber protein" refers to a chimeric fiber protein comprising a portion or the entirety of a fiber protein derived from Ad35 and a portion or the entirety of a fiber protein derived from Ad5, and its specific structure is not particularly limited as long as it is capable of binding to CD46. For example, it may be a chimeric fiber protein comprising the knob portion and the shaft portion of a fiber protein derived from Ad35 and the tail portion of a fiber protein derived from Ad5. Further, one or more amino acids may be deleted, substituted, or added in such a protein, as long as it is capable of binding to CD46. Preferred examples of Ad5/35 chimeric fiber proteins include a protein comprising the amino acid sequence of SEQ ID NO: 14. More preferred examples include a protein consisting of the amino acid sequence of SEQ ID NO: 14. The base sequence (H) is not limited as long as it is a base sequence encoding such a polypeptide.

Specifically, examples of the base sequence (H) include the following (h-1) and (h-2).
(h-1): A base sequence consisting of the base sequence of SEQ ID NO: 8
(h-2): A base sequence consisting of a base sequence having a sequence identity of 85% or more to the base sequence of (h-1), and encoding a protein that can bind to CD46

The sequence identity may be 85, 86, 87, 88, 89, 90, 91, 92, 93, 94, 95, 96, 97, 98, or 99% or more, preferably 90% or more, more preferably 95% or more, and particularly preferably 99% or more.

### 2-10. Others

The nucleic acid of the present disclosure may further comprise other sequences in addition to the sequences described above. Examples of other sequences include signal peptide-encoding sequences, promoter sequences, enhancer sequences, repressor sequences, insulator sequences, origins of replication, and drug resistance gene-encoding sequences. The nucleic acid of the present disclosure may be a linear nucleic acid or a cyclic nucleic acid.

The nucleic acid of the present disclosure can be produced by a known method or a method easily conceivable from a known method. For example, it can be produced by genetic engineering techniques such as PCR, restriction enzyme digestion, DNA ligation techniques, and in vitro transcription techniques. The nucleic acid of the present disclosure may be produced through chemical synthesis.

### 3. Use

The oncolytic adenoviral vector of the present disclosure can exhibit an excellent anticancer effect. Thus, the technique of the present disclosure is suitably used for preventing, ameliorating, and treating cancer, for example.

In the present disclosure, the term "preventing cancer" includes preventing the development of cancer by applying the treatment to a subject before the subject is diagnosed with cancer, and preventing cancer metastasis by applying the treatment to tissues in which cancer has not been detected in a subject diagnosed with cancer. In the present disclosure, the terms "ameliorating cancer" or "treating cancer" include, when applied to a subject diagnosed with cancer, inhibiting the progression of cancer, stopping the progression of cancer, inhibiting the proliferation of cancer cells, killing cancer cells, reducing the number of cancer cells, reducing tumor volume, and preventing cancer metastasis. Further, in the present disclosure, the term "anticancer" includes the prevention of cancer and its effects, the amelioration of cancer and its effects, and the treatment of cancer and its effects.

In the present disclosure, the term "cancer" encompasses cancer, neoplasms, and malignant tumors. Examples of cancers include bladder cancer, breast cancer, prostate cancer, malignant melanoma, renal cancer, mesothelioma, intraperitoneally disseminated cancer, lung cancer, colorectal cancer, gastric cancer, soft tissue sarcoma, osteosarcoma, brain tumor, neuroblastoma, leukemia, lymphoma, liver cancer, retinoblastoma, ciliary body tumor, basal cell carcinoma, squamous cell carcinoma, malignant soft tissue tumor, chondrosarcoma, ovarian cancer, endometrial cancer, and cervical cancer. The cancer to which the technique of the present disclosure may be applied is not particularly limited, and is particularly suitably applied to at least one selected from the group consisting of bladder cancer, breast cancer, and prostate cancer.

Examples of subjects to which the technique of the present disclosure may be applied include humans and non-human mammals (e.g., rats, mice, rabbits, cattle, pigs, dogs, cats, sheep, and monkeys), among which humans are preferred.

### 4. Application Method

The application method of the technique of the present disclosure is not particularly limited as long as a desired effect is achieved. For example, the oncolytic adenoviral vector of the present disclosure or a composition containing the same may be directly administered into a lesion by injection, or may be administered by injection or infusion intravenously, intra-arterially, intraperitoneally, intramuscularly, subcutaneously, intrapleurally, or the like, and may also be administered by perfusion via a catheter. Administration by injection is particularly preferred.

The frequency of application of the technique of the present disclosure is not particularly limited. For example, the technique may be applied one or more times per day, one or more times per week, one or more times per month, or one or more times per year. The application period of the technique of the present disclosure is also not particularly limited as long as the effect of the present disclosure is achieved. Further, the dose of the oncolytic adenoviral vector of the present disclosure is also not particularly limited as long as the effect of the present disclosure is achieved. The application frequency, application period, and dose may be appropriately adjusted by a person skilled in the art depending on the conditions of the subject, the course of treatment, and the like.

The technique of the present disclosure may be optionally combined with other pharmaceutical compositions and/or therapy methods, etc. that are applied to subjects with cancer. When the technique of the present disclosure is combined with other pharmaceutical compositions and/or therapy methods, etc., they may be applied to a subject simultaneously, or each may be applied separately at any given time.

In the technique of the present disclosure, the oncolytic adenoviral vector of the present disclosure may be mixed with a pharmaceutically acceptable base, carrier, excipient, diluent, solubilizer, emulsifier, preservative, pH adjuster, adjuvant, chelating agent, etc. These components may be used alone or in a combination of two or more.

Examples of preservatives include parabens such as methylparaben, ethylparaben, propylparaben, and butylparaben; sodium benzoate; phenoxyethanol; and alkyl diaminoethyl glycine hydrochloride. These preservatives may be used alone or in a combination of two or more.

Examples of pH adjusters include citric acid, phosphoric acid, malic acid, pyrophosphoric acid, lactic acid, tartaric acid, glycerophosphoric acid, acetic acid, nitric acid, chemically acceptable salts of these acids, sodium hydroxide, and potassium hydroxide. These pH adjusters can be used alone or in a combination of two or more such that the pH of the composition containing the oncolytic adenoviral vector of the present disclosure is in the range from 4 to 8, preferably from 5 to 7.

The method for preparing the composition containing the oncolytic adenoviral vector of the present disclosure is not particularly limited, as long as the effect of the present disclosure is achieved. For example, the composition can be prepared in accordance with a method known in the art. More specifically, for example, the composition can be prepared by mixing the oncolytic adenoviral vector of the present disclosure and other components with sterilized distilled water.

### 5. Composition

The present disclosure also encompasses an anticancer composition containing the oncolytic adenoviral vector of the present disclosure. Such an anticancer composition is sometimes referred to as "the anticancer composition of the present disclosure." The matters described in the sections from "1. Oncolytic Adenoviral Vector" to "4. Application Method" are incorporated into the anticancer composition of the present disclosure.

### 6. Proposed Mechanism

Without wishing to be bound by theory, the protein encoded by the base sequence (I) or the base sequence (III) is considered to exhibit an anticancer effect through the following mechanism of action. The fusion protein encoded by the base sequence (I) or the base sequence (III) comprises (i) a protein portion having a CD44 extracellular function, (ii) a protein portion having a Notch core region function, and (iii) a protein portion having an HIF-3α4 function. Thus, (i) can function as a decoy receptor, and a signal can be transmitted to (iii) via (ii). This becomes more effective when (i) is fused upstream of (ii). HIF-1α activated in tumors can be inhibited by fusing (iii) downstream of (ii). Presumably, this achieves an anticancer effect based on multiple mechanisms of action.

Presumably, the base sequence (II) contributes to the efficient exertion of the anticancer effect by the oncolytic adenoviral vector of the present disclosure via the following mechanism of action. As described above, a gene or protein encoded by a base sequence placed under the control of the COX-2 promoter may exhibit increased expression in cells in an inflammatory state or in cancer cells. Thus, E1A protein and E1B protein under the control of the COX-2 promoter are considered to exhibit increased expression in cancer cells. Expression of E1A protein and E1B protein causes replication and propagation of the oncolytic adenoviral vector of the present disclosure in the cancer cells, resulting in lysis and death of the cancer cells. Further, the oncolytic adenoviral vectors of the present disclosure that have propagated within the cancer cells are released from the dead cancer cells, and can infect other cancer cells present in the vicinity. Thereafter, propagation of the oncolytic adenoviral vector of the present disclosure, lysis and death of cancer cells, release of the oncolytic adenoviral vectors, and infection of neighboring cancer cell can happen repeatedly.

Further, presumably, replication and propagation of the oncolytic adenoviral vector of the present disclosure within the cancer cells increases the abundance of proteins encoded by the base sequence (I) or the base sequence (III) in a cancer cell-specific manner, as described above. In other words, in the technique of the present disclosure, presumably, incorporation of the base sequence (I) or the base sequence (III) into the oncolytic adenoviral vector results in mutual association between the anticancer effect of the oncolytic adenoviral vector and the anticancer effect of the fusion protein encoded by the base sequence (I) or the base sequence (III), whereby a synergistically enhanced anticancer effect is exerted in a cancer cell-specific manner.

As is clear from the proposed mechanism described above, the technique of the present disclosure is predicted to exert an excellent anticancer effect particularly on COX-2 positive cancer cells. Thus, the technique of the present disclosure is particularly suitably used for COX-2 positive cancer cells. In the present disclosure, the term "COX-2 positive" means that the COX-2 expression level is increased in cells in an inflammatory state or in cancer cells, as compared to the COX-2 expression level in normal cells that are not in an inflammatory state. Whether a given cell is COX-2 positive can be analyzed by conventionally known methods or methods that can be readily conceived from such known methods. Examples of such methods include reverse transcription quantitative PCR (RT-qPCR), RNA-seq, Western blotting, and immunohistochemical staining.

Although the reason is not clear, as described in Test Example 2.5, when the CD44/Notch/HIF-3α4 fusion gene and the E1A/E1B gene were arranged in reverse, the expression of the CD44/Notch/HIF-3α4 fusion gene was not detected in either transcription or translation. In contrast, as described in Test Example 2.6 and Test Example 2.7, when the CD44/Notch/HIF-3α4 fusion gene and the E1A/E1B gene were arranged in the same orientation, a significant level of expression of the CD44/Notch/HIF-3α4 fusion gene was detected. Thus, in the technique of the present disclosure, it is desirable that the base sequence (I) or the base sequence (III), and the base sequence (II) are arranged in the same orientation.

In the present specification, the term "comprising" includes "consisting essentially of" and "consisting of." Further, the present disclosure includes all of any combinations of the features described in the present specification

In addition, the various characteristics (properties, numerical values, functions, etc.) described in each embodiment of the present disclosure described above may be combined in any way in specifying the main subjects included in the present disclosure. In other words, the present disclosure includes all the main subjects comprising all combinations of the combinable characteristics described in the present specification.

### Examples

Embodiments of the present disclosure are described in more detail below while providing examples; however, the embodiments of the present disclosure are not limited to the following examples.

### 1. Experimental Method

### 1.1. Construction of CRAd-synNotch (Forward/Reverse)

In this Example, the base sequence actually designed with a structure in which (A) a base sequence encoding a protein having a CD44 extracellular function, (B) a base sequence encoding a protein having a Notch core region function, and (C) a base sequence encoding a protein having an HIF-3α4 function are linked in the order of (A)-(B)-(C) may be referred to as "CD44/Notch/HIF-3α4 fusion gene." The CD44/Notch/HIF-3α4 fusion gene is a preferred embodiment of the base sequence (I) or the base sequence (III) in the technology of the present disclosure.

In this Example, a recombinant oncolytic adenoviral vector in which a COX-2 promoter region is placed upstream of E1A gene and E1B gene and the CD44/Notch/HIF-3α4 fusion gene is inserted may be referred to as "CRAd-synNotch (forward/reverse)." In CRAd-synNotch (forward), the CD44/Notch/HIF-3α4 fusion gene is arranged in the same orientation (forward) as the COX-2 promoter region, E1A gene, and E1B gene. On the other hand, in CRAd-synNotch (reverse), the CD44/Notch/HIF-3α4 fusion gene is arranged in the reverse orientation relative to the COX-2 promoter region, E1A gene, and E1B gene. The method for constructing CRAd-synNotch (forward/reverse) is described below.

The pAd1127-04, pAd1128, pAd1129-06, and pAd1130-04 plasmids included in AdenoQuick 2.0 Kit (O.D.260 Inc.) were used as the vector DNA used in this Example. Cosmid Construction Kit-2 (O.D.260 Inc.) was used for the construction of a cosmid vector.

### 1.1.1 Amplification and Cloning Enhancement of COX-2 Promoter Region

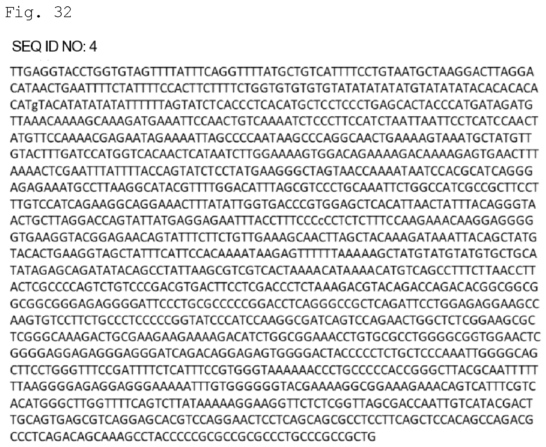

Using 25 ng of pDrive-hCOX2 (InvivoGen) as a template, PCR was performed with KOD-Plus-Neo (Toyobo Co., Ltd.) to amplify the COX-2 promoter region. The composition of the PCR reaction solution and the primers used for PCR are shown in the tables below. In addition, the base sequence of the amplified COX-2 promoter region (SEQ ID NO: 4) is shown in Fig. 32.

**Table 1**

| Composition of PCR Reaction Solution | |
|---|---|
| KOD Plus neo buffer | 2.5*µ*l |
| dNTPS (2 mM) | 2.5*µ*l |
| MgSO4 (25mM) | 1.5*µ*l |
| Forward primer (10*µ*M) | 0.75*µ*l |
| Reverse primer (10*µ*M) | 0.75*µ*l |
| pDrive-hCOX2 | 25 ng |
| KOD Plus neo polymerase | 0.5*µ*l |
| Sterile purified water | Up to 25*µ*l |

**Table 2**

| Primer List | | |
|---|---|---|
| Primer name | Sequence (5'-3') | |
| Cox-2_infusion_Forward | AGCGGCCGCTGTCGATTGAGGTACCTGGTGTAGTT | SEQ ID NO:16 |
| Cox-2_infusian_Reverse | TAGTGATATCGAATTACAGCGGCGGGCAGGGCG | SEQ ID NO:17 |

5 µl of the PCR product and 2 µl of Cloning Enhancer (TaKaRa) were reacted at 37°C for 20 minutes, and then enzyme inactivation was performed by heating at 80°C for 15 minutes.

### 1.1.2 Restriction Enzyme Treatment of pAd1127-04 Plasmid

The pAd1127-04 plasmid included in AdenoQuick 2.0 Kit (O.D.260 Inc.) was digested using SalI (TaKaRa) and EcoRI (TaKaRa) restriction enzymes, and after agarose gel electrophoresis, purification was performed using Wizard (trademark) SV Gel and PCR Clean-Up System (Promega Corporation). The composition of the restriction enzyme-treated reaction solution is shown in the table below.

**Table 3**

| Composition of Restriction Enzyme-Treated Reaction Solution of pAd1127-04 | |
|---|---|
| pAd1127-04 | 1.0*µ*l |
| Sall (15U/*µ*l) | 0.5*µ*l |
| EcoRI (15U/*µ*l) | 0.5*µ*l |
| H buffer | 2.0*µ*l |
| Sterile purified water | Up to 20*µ*l |

### 1.1.3 Insertion of COX-2 Promoter Region into pAd1127-04 Plasmid and Transformation into E. coli

To ligate the COX-2 promoter region amplified in item 1.1.1 to pAd1127-04, an In-Fusion reaction (50°C, 15 minutes) was performed using In-Fusion (trademark) HD Cloning Kit (TaKaRa). The reaction solution was used to transform *Escherichia coli* DH5α Competent Cells (TaKaRa). The transformation was performed by a method according to the manual of the kit.

The transformed *E. coli* DH5α Competent Cells were seeded on LB agar medium (Nacalai Tesque) supplemented with 25 µg/ml kanamycin, and cultured at 37°C overnight. After the culture, the colonies grown on the LB agar medium were used as templates for insert check PCR using KOD-Plus-Neo, and it was confirmed by agarose gel electrophoresis whether the transformation was successful.

Colonies confirmed to have undergone successful transformation were subcultured in LB liquid medium supplemented with 25 µg/ml kanamycin, and the plasmid was extracted from the culture solution using a PureYield (trademark) Plasmid Midiprep System (Promega Corporation). The sequence in the extracted plasmid was confirmed using a DNA sequencer.

### 1.1.4 Insertion of CD44/Notch/HIF-3α4 Fusion Gene into pAd1129-06 and Transformation into E. coli

The construction of a plasmid carrying the CD44/Notch/HIF-3α4 fusion gene was outsourced to Gene Universal. The plasmid carrying the fusion gene and pAd1129-06 plasmid (O.D.260 Inc.) were each treated with EcoRI (TaKaRa) restriction enzyme, followed by agarose gel electrophoresis and purification. The pAd1129-06 plasmid is an oncolytic adenoviral vector. The compositions of the restriction enzyme-treated reaction solutions are shown in the tables below.

**Table 4**

| Composition of Restriction Enzyme-Treated Reaction Solution of pAd1129-06 | |
|---|---|
| pAd1129-06 | 1.0*µ*l |
| EcoRI (15U/*µ*l) | 1.0*µ*l |
| H buffer | 2.0*µ*l |
| Sterile purified water | Up to 20*µ*l |

**Table 5**

| Composition of Restriction Enzyme-Treated Reaction Solution of Fusion Gene-Bearing Plasmid | |
|---|---|
| Fusion Gene-Bearing Plasmid | 1.0*µ*g |
| EcoRI (15U/*µ*l) | 1.0*µ*l |
| H buffer | 2.0*µ*l |
| Sterile purified water | Up to 20*µ*l |

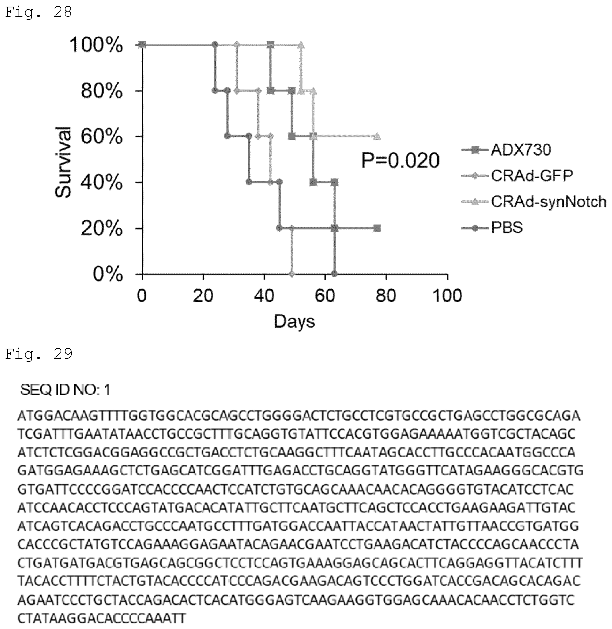

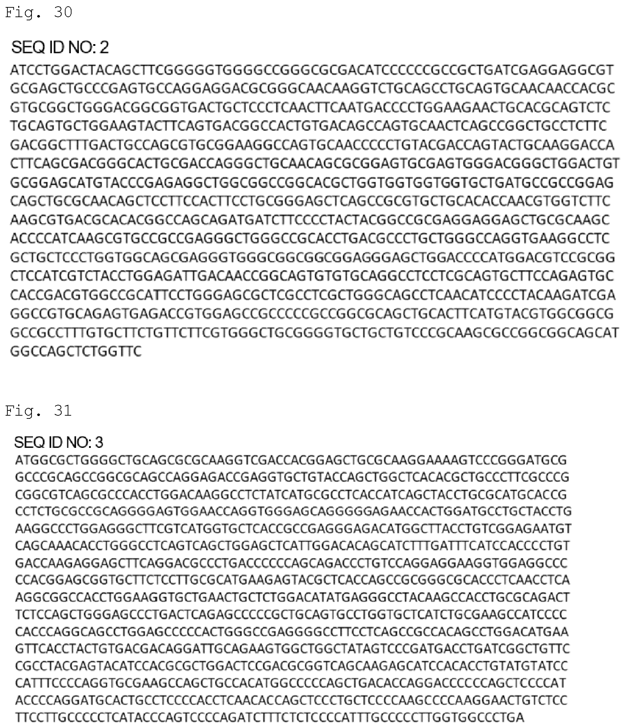

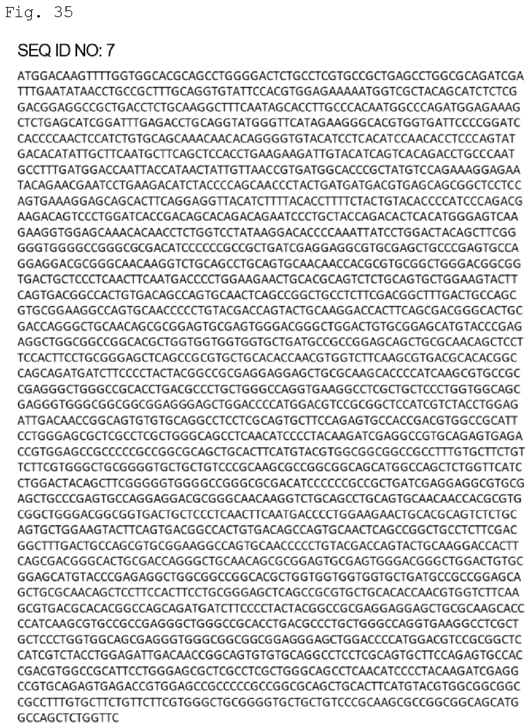

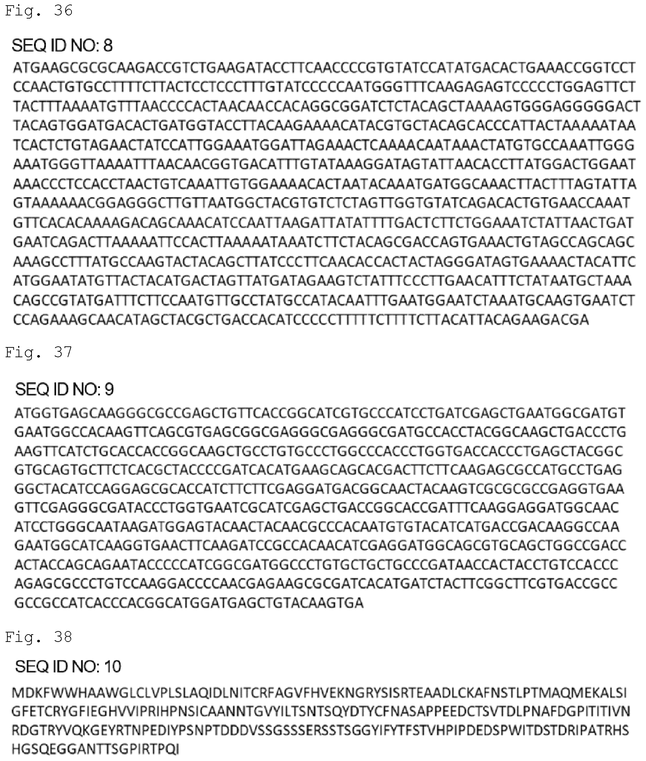

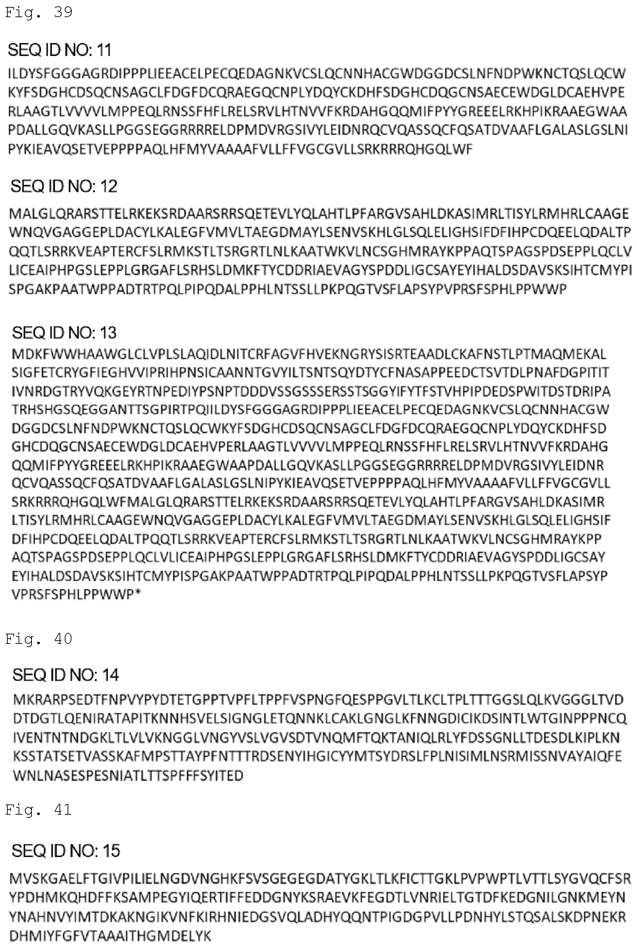

Fig. 35 shows the sequence of the CD44/Notch/HIF-3α4 fusion gene (SEQ ID NO: 7). Figs. 29 to 31 show the base sequence encoding a protein having a CD44 extracellular function (SEQ ID NO: 1), the base sequence encoding a protein having a Notch core region function (SEQ ID NO: 2), and a base sequence encoding a protein having a HIF-3α4 function (SEQ ID NO: 3), which are contained in the CD44/Notch/HIF-3α4 fusion gene, respectively. Furthermore, Fig. 39 shows the amino acid sequence of the protein (SEQ ID NO: 13) encoded by the base sequence of SEQ ID NO: 7, and Fig. 38 shows the amino acid sequences of the proteins (SEQ ID NOs: 10 to 12) encoded by the base sequences of SEQ ID NOs: 1 to 3.

The purified plasmids and Ligation Mix (TaKaRa) were mixed, and a ligation reaction was performed (16°C, 30 minutes). The reaction solution was used to transform *Escherichia coli* DH5α Competent Cells (TaKaRa). The transformation was performed by a method according to the manual of the kit.

The transformed *E. coli* DH5α Competent Cells were seeded on LB agar medium (Nacalai Tesque) supplemented with 100 µg/ml ampicillin, and cultured at 37°C overnight. After the culture, the colonies grown on the LB agar medium were used as templates for insert check PCR using KOD-Plus-Neo, and it was confirmed by agarose gel electrophoresis whether the transformation was successful.

Colonies confirmed to have undergone successful transformation were subcultured in LB liquid medium supplemented with 100 µg/ml ampicillin, and the plasmid was extracted from the culture solution using a PureYield (trademark) Plasmid Midiprep System (Promega Corporation). The sequence in the extracted plasmid was confirmed using a DNA sequencer.

### 1.1.5 Construction of Recombinant Cosmid Vector and Transformation into E. coli

The pAd1127-04 plasmid into which the COX-2 promoter region was inserted in item 1.1.3 and the pAd1129-06 plasmid into which the CD44/Notch/HIF-3α4 fusion gene was inserted in item 1.1.4, as well as pAd1128 (O.D.260 Inc.) and pAd1130-04 (O.D.260 Inc.) were each digested using SfiI (New England Biolabs) restriction enzyme. After each reaction solution was subjected to agarose gel electrophoresis, the DNA fragments were purified.

The purified DNA fragments and T4 DNA Ligase (TaKaRa) were mixed, and a ligation reaction was performed (16°C, overnight). 2.0 µl of the reaction solution and 5.0 µl of λ packaging extract included in Cosmid Construction Kit-2 were reacted at 30°C for 1 hour. After the reaction, 100 µl of SM medium included in the kit and 5.0 µl of chloroform (Nacalai Tesque) were added, and the mixture was tapped and then centrifuged (15000 rpm, 1 minute). 20 µl of the centrifugation supernatant and 20 µl of a culture solution of *E. coli* TOP10 Competent Cells (Invitrogen) cultured in LB liquid medium supplemented with 0.2% maltose and 10 mM MgSO₄ were mixed and reacted at 37°C for 30 minutes. After the reaction, seeding was performed on LB agar medium (Nacalai Tesque) supplemented with 50 µg/ml ampicillin and 25 µg/ml kanamycin, followed by culture at 37°C overnight. After the culture, the colonies grown on the LB agar medium were used as templates for colony PCR, and colonies carrying a cosmid vector in which the CD44/Notch/HIF-3α4 fusion gene was linked in the same orientation (forward) as the COX-2 promoter, and colonies carrying a cosmid vector in which the CD44/Notch/HIF-3α4 fusion gene was linked in the reverse orientation relative to the COX-2 promoter were selected. Each selected colony was subcultured in LB liquid medium supplemented with 50 µg/ml ampicillin and 25 µg/ml kanamycin, and the cosmid vectors were extracted from the culture solutions using a PureYield (trademark) Plasmid Midiprep System (Promega Corporation).

In this step, four samples of the cosmid vector in which the CD44/Notch/HIF-3α4 fusion gene was linked in the same orientation (forward) as the COX-2 promoter were obtained (a sample derived from a single colony on the LB agar medium containing ampicillin and kanamycin described above is counted as one sample). In addition, one sample of the cosmid vector in which the CD44/Notch/HIF-3α4 fusion gene was linked in the reverse orientation relative to the COX-2 promoter was obtained.

### 1.1.6 Construction of Conditionally Replicative Recombinant Adenoviral Vector by Transformation of HEK293 Cells with Recombinant Cosmid

Each recombinant cosmid vector DNA prepared in item 1.1.5 was digested using PacI (New England Biolabs) restriction enzyme. After the restriction enzyme treatment, nucleic acid purification was performed by phenol-chloroform extraction and ethanol precipitation, and the purified nucleic acid was dissolved in 30 µl of sterile purified water. 1 µl of the resulting solution was used for agarose gel electrophoresis, and digestion with PacI restriction enzyme was confirmed.

10 µg of the PacI-digested cosmid was mixed with Lipofectamine (trademark) 2000 (Invitrogen) and Opti-MEM (trademark) I Reduced Serum Medium (Gibco), and HEK293 cells (National Institutes of Biomedical Innovation, Health and Nutrition) cultured to confluence in a 60 mm tissue culture Petri dish were subjected to lipofection.

After the lipofection, the cultured cells were collected and seeded on 16 plates of Collagen-Coated Microplate 96 Well with Lid Collagen Type I (AGC TECHNO GLASS) per clone. 4 days, 8 days, and 16 days after seeding, 50 µl of Dulbecco's modified Eagle's medium (D-MEM; FUJIFILM Wako Pure Chemical Corporation) containing 5% fetal bovine serum (FBS; Sigma Aldrich) was added to each well, and the cells were cultured with daily observation until all of the cells exhibited a complete cytopathic effect.

For one clone obtained by using the cosmid vector obtained in item 1.1.5 in which the CD44/Notch/HIF-3α4 fusion gene was linked in the reverse orientation relative to the COX-2 promoter, only one well in which the cells had exhibited a complete cytopathic effect was collected into a 1.5 ml tube 22 days after seeding. Thereafter, freezing with liquid nitrogen and thawing with a 37°C warm bath were repeated 6 times.

Cytopathic effects were confirmed in three of the samples in which HEK293 cells were transformed with each of the four samples of the cosmid vector obtained in item 1.1.5 in which the CD44/Notch/HIF-3α4 fusion gene was linked in the same orientation (forward) as the COX-2 promoter, using the method described above. Three wells in which the cells had exhibited a complete cytopathic effect were collected into 1.5 ml tubes 36 days, 38 days, and 39 days after seeding (which may be referred to as "clones 1 to 3"), respectively. Thereafter, freezing with liquid nitrogen and thawing with a 37°C warm bath were repeated 6 times.

After the last freeze-thaw, each clone was subjected to centrifugation (5000 rpm, 5 minutes, 4°C) to collect the supernatant. The supernatant was stored as a primary virus solution. The above method is a modified version of the method described in *Kumikae Adenouirusu Sakusei Manyuaru: Kanzencho Genomu Donyuho* [Manual for Recombinant Adenovirus Production: Method for Introducing Full-Length Genome] (Gene Engineering Division, BioResource Center, RIKEN).

### 1.1.7 Purification of High-Titer Recombinant Adenoviral Vector

HEK293 cells (National Institutes of Biomedical Innovation, Health and Nutrition) were cultured to 80% to 100% confluence in Collagen-Coated Microplate 24 Well with Lid Collagen Type I (AGC TECHNO GLASS). After the culture, the medium was removed, and a mixture of 0.1 ml of Dulbecco's modified eagle medium (D-MEM; FUJIFILM Wako Pure Chemical Corporation) containing 5% FBS and 10 µl of the primary virus solution prepared in item 1.1.6 was added per well. To infect the HEK293 cells with the virus, the plate was slowly shaken several times in an incubator (37°C, 5% CO₂), and this operation was performed a total of four times, every 15 minutes. After 1 hour of infection, 0.4 ml of 5% FBS-E-MEM was added to each well, and the cells were cultured for 3 days. After the culture, cells that had exhibited a complete cytopathic effect were collected together with the culture solution. For the collected cells, freezing with liquid nitrogen and thawing with a 37°C warm bath were repeated 6 times, as with the primary virus solution. After the last freeze-thaw, centrifugation (5000 rpm, 5 minutes, 4°C) was performed, and the collected supernatant was stored as a secondary virus solution.

Next, HEK293 cells were cultured to 80 to 100% confluence in a 60 mm tissue culture Petri dish, and 0.4 ml of 5% FBS-D-MEM and 100 µl of the secondary virus solution were added to infect the cells with the virus. For infection, the plate was slowly shaken several times in an incubator (37°C, 5% CO₂), and this operation was performed a total of four times, every 15 minutes. After 1 hour of infection, 2.5 ml of 5% FBS-D-MEM was further added, and the cells were cultured for 3 days. After the culture, it was confirmed that the cells exhibited a complete cytopathic effect, and the cells were collected together with the culture solution. For the collected cells, freezing with liquid nitrogen and thawing with a 37°C warm bath were repeated 6 times, as with the primary virus solution. After the last freeze-thaw, centrifugation (3000 rpm, 10 minutes, 4°C) was performed, and the collected supernatant was stored as a tertiary virus solution.

### 1.1.8 Mass Culture and Purification of Recombinant Adenoviral Vector

Mass culture and purification of the recombinant adenoviral vector constructed in item 1.1.7 were performed. The specific method is described below.

HEK293 cells were seeded to 80 to 100% confluence in 10 Corning (trademark) 225 cm² flasks (Corning). 5 ml of 5% FBS-D-MEM and 500 µl of the tertiary virus solution prepared in item 1.1.7 were added to the HEK293 cells to infect the cells with the virus. For infection, each flask was slowly shaken several times in an incubator (37°C, 5% CO₂), and this operation was performed a total of four times, every 15 minutes. After 1 hour of infection, 30 ml of 5% FBS-D-MEM was added, and the cells were cultured for 2 days. After the culture, it was confirmed that all of the cells exhibited a complete cytopathic effect, and the cells were collected together with the culture solution. After collection, centrifugation (3000 rpm, 10 minutes, 4°C) was performed, the supernatant was removed, fresh 10% FBS-D-MEM was added, and cell pellets were resuspended. Then, freezing with liquid nitrogen and thawing with a 37°C warm bath were repeated 6 times.

After the last freeze-thaw, centrifugation (3000 rpm, 10 minutes, 4°C) was performed, and the cells were collected again. Only the supernatant was collected and purified by the ultracentrifugation method described below.

Using a 5 ml syringe with a needle, 3 ml of sterile 2.2 M CsCl solution was poured from the bottom into a Beckman tube (Beckman Coulter), and then 3 ml of sterile 4.0 M CsCl solution was slowly added from the bottom of the tube. Using a 5 ml syringe with a needle, 2.5 ml of the centrifugation supernatant described above was gently layered on top of the 2.2 M CsCl solution described above. The compositions of the solutions are shown in the tables below.

**Table 6**

| Composition of 2.2 M CsCl | |
|---|---|
| CsCl | 38 9 |
| 10 mM HEPES | 80ml |
| 10 mM HEPES | Up to 100ml |

**Table 7**

| Composition of 4.0 M CsCl | |
|---|---|
| CsCl | 67 g |
| 10 mM HEPES | 80ml |
| 10 mM HEPES | Up to 100ml |

**Table 8**

| Composition of 10 mM HEPES (pH 7.4) | |
|---|---|
| HEPES | 1.192 g |
| Ultrapure water | 400ml |
| Ultrapure water | Up to 500ml |

The tube was sealed using a Beckman Coulter cordless tube topper (Beckman Coulter) and subjected to ultracentrifugation (30000 rpm, 4°C, 18 hours). After the ultracentrifugation, a 23G needle attached to a 5 ml syringe was inserted slightly below the intermediate layer (band) observed in the tube, and 1.5 ml of liquid was collected so as to collect as much of the band as possible. The collected liquid was stored in a 2 ml tube.

### 1.1.9 Measurement of Titer of Recombinant Adenoviral Vector

The titer of the recombinant adenoviral vector constructed in item 1.1.8 was measured. The specific method is described below.

HEK293 cells were seeded in a 12-Well Cell Culture Plate Flat Bottom (Corning), and the virus solution mass-cultured and purified in item 1.1.8 that was diluted from 100-fold to 10 million-fold in 10-fold increments was added dropwise to each well, and the cells were cultured for 2 days. After the culture, the entire medium was removed, the cells were slightly dried, and then 1 ml of methanol (FUJIFILM Wako Pure Chemical Corporation) at -20°C was added dropwise and incubated (-20°C, 10 minutes). After the incubation, the entire methanol was removed, and the resultant was washed three times with Dulbecco's PBS(-) "Nissui" (PBS; Nissui Pharmaceutical Co., Ltd.) containing 1% bovine serum albumin (BSA; Sigma Aldrich).

After washing, titer measurement was performed using an Adeno-X (trademark) Rapid Titer Kit (TaKaRa). Specifically, 0.5 ml of Mouse Anti-Hexon Antibody diluted 1000-fold with PBS containing 1% BSA was added dropwise and incubated (37°C, 1 hour) while shaking with a shaker. After the reaction, the resultant was washed three times with PBS containing 1% BSA, and 0.5 ml of HRP conjugate Rat Anti-Mouse Antibody diluted 500-fold with PBS containing 1% BSA was added dropwise and incubated (37°C, 1 hour) while shaking with a shaker. After the reaction, the resultant was washed three times with PBS containing 1% BSA, and 0.5 ml of 10× DAB Substrate diluted 10-fold with 1× Stable Peroxidase Buffer was added dropwise and incubated (room temperature, 10 minutes). After the reaction, the entire reaction liquid was removed, 1 ml of PBS was added, and the titer was determined by observing the stained cells under a microscope. The above procedure was performed by a method according to the manual of the Adeno-X (trademark) Rapid Titer Kit (TaKaRa).

### 1.2 Construction of CRAd-GFP

As a negative control for CRAd-synNotch (forward) constructed in item 1.1, a conditionally replicative adenoviral vector was constructed by inserting AcGFP1 (which may be simply referred to as "GFP" in the present disclosure) gene in place of the CD44/Notch/HIF-3α4 fusion gene of CRAd-synNotch (forward). This adenoviral vector may be referred to as "CRAd-GFP." Fig. 37 shows the base sequence of GFP gene used in this Example (SEQ ID NO: 9), and Fig. 41 shows the amino acid sequence (SEQ ID NO: 15) of the protein encoded by the base sequence of SEQ ID NO: 9. The specific method for constructing CRAd-GFP is described below.

A pAd1129-06 plasmid (O.D.260 Inc.) and a pAcGFP1 Vector (TaKaRa) were each digested with SalI (TaKaRa) and EcoRI (TaKaRa) restriction enzymes, followed by agarose gel electrophoresis and then purification. The compositions of the restriction enzyme-treated reaction solutions are shown in the tables below.

**Table 9**

| Composition of Restriction Enzyme-Treated Reaction Solution of pAd1129-06 | |
|---|---|
| pAd1129-06 | 1 µg |
| SalI (15U/µl) | 0.5µl |
| EcoRI (150/µl) | 0.5µl |
| H buffer | 2.0µl |
| Sterile purified water | Up to 20µl |

**Table 10**

| Composition of Restriction Enzyme-Treated Reaction Solution of pAcGFP1 Vector | |
|---|---|
| pAd1129-06 | 1 µg |
| SaiI (15U/µl) | 0.5 µl |
| EcoRI (150/µl) | 0.5 µl |
| H buffer | 2.0 µl |
| Sterile purified water | Up to 20µl |

The purified nucleic acid fragments and Ligation Mix (TaKaRa) were mixed, and a ligation reaction was performed (16°C, 30 minutes). The reaction solution was used to transform *Escherichia coli* DH5α Competent Cells (TaKaRa). The transformation was performed by a method according to the manual of the kit.

The transformed *E. coli* DH5α Competent Cells were seeded on LB agar medium (Nacalai Tesque) supplemented with 100 ug/ml ampicillin, and cultured at 37°C overnight. After the culture, the colonies grown on the LB agar medium were used as templates for insert check PCR using KOD-Plus-Neo, and it was confirmed by agarose gel electrophoresis whether the transformation was successful.

Colonies confirmed to have undergone successful transformation were subcultured in LB liquid medium supplemented with 100 µg/ml ampicillin, and the plasmid was extracted from the culture solution using a PureYield (trademark) Plasmid Midiprep System (Promega Corporation). The sequence in the extracted plasmid was confirmed using a DNA sequencer. The primers used for sequencing are shown in the table below.

**Table 11**

| Sequencing Primer List | | |
|---|---|---|
| Primer name | Sequence (5'→3') | |
| pAd1129-06+CD44 sense primer | CTAGTTGAGCGGGACAGGG | SEQ ID NO:18 |
| 1129-06-Nhe1-EcoR1-R | AGAGCTGGGAGGAGGGCA | SEQ ID NO: 19 |

Except for item 1.1.4, the subsequent procedures were performed in the same manner as in item 1.1 to prepare CRAd-GFP.

### 1.3 Various Adenoviral Vectors

The adenoviral vectors used in this Example are as follows: CRAd-synNotch (forward) and CRAd-synNotch (reverse) constructed in item 1.1; CRAd-GFP constructed in item 1.2; the ADX730 non-replicating type 5 recombinant adenoviral vector described in PTL 1, in which the CD44/Notch/HIF-3α4 fusion gene is incorporated into the E1 region; wild-type adenovirus type 5 (wtAd), which exhibits COX-2-independent propagation; a recombinant adenoviral vector in which LacZ gene is incorporated into the E1 region (Ad-LacZ).

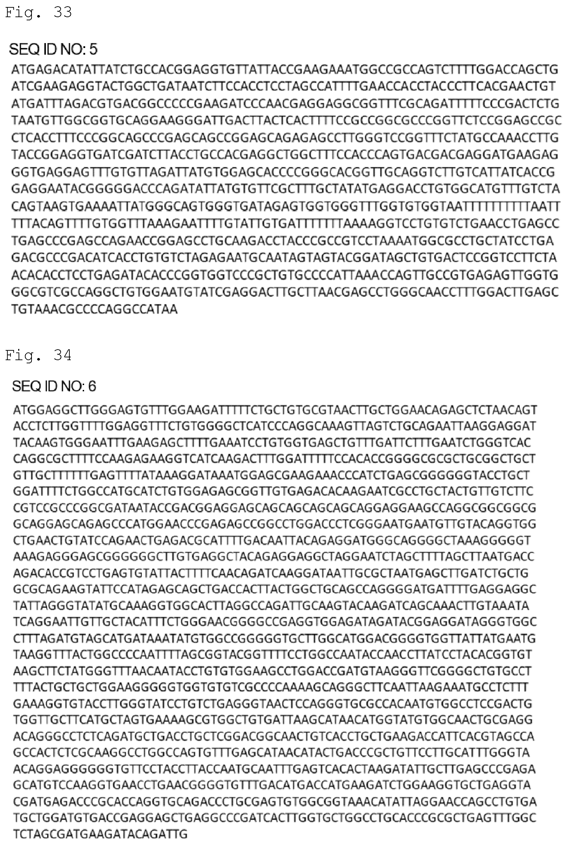

As described above, CRAd-synNotch (forward/reverse) is a recombinant oncolytic adenoviral vector in which the COX-2 promoter region is placed upstream of E1A gene and E1B gene, and the CD44/Notch/HIF-3α4 fusion gene is inserted. In CRAd-synNotch (forward), the CD44/Notch/HIF-3α4 fusion gene is arranged in the same orientation (forward) as the COX-2 promoter region, E1A gene, and E1B gene. On the other hand, in CRAd-synNotch (reverse), the CD44/Notch/HIF-3α4 fusion gene is arranged in the reverse orientation relative to the COX-2 promoter region, E1A gene, and E1B gene. Figs. 33 and 34 show the base sequence of E1A gene (SEQ ID NO: 5) and the base sequence of E1B gene (SEQ ID NO: 6) used in this Example, respectively.

CRAd-GFP is a recombinant oncolytic adenoviral vector in which GFP gene is inserted in place of the CD44/Notch/HIF-3α4 fusion gene of CRAd-synNotch (forward). As described in PTL 1, ADX730 is a recombinant adenoviral vector in which the CD44/Notch/HIF-3α4 fusion gene is inserted, but which does not contain E1A gene or E1B gene controlled by the COX-2 promoter. wtAd is an adenoviral vector that does not have the CD44/Notch/HIF-3α4 fusion gene, or the E1A gene or E1B gene, both of which are controlled by the COX-2 promoter. Ad-LacZ is a recombinant adenoviral vector in which LacZ gene is inserted in place of the CD44/Notch/HIF-3α4 fusion gene of ADX730.

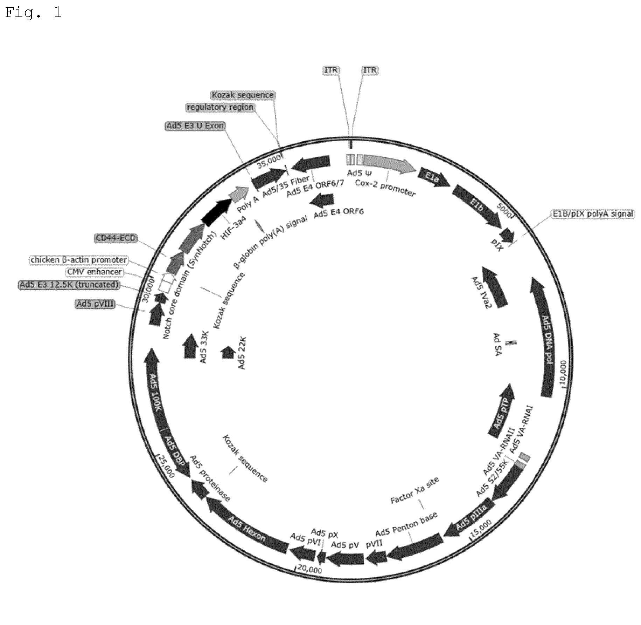

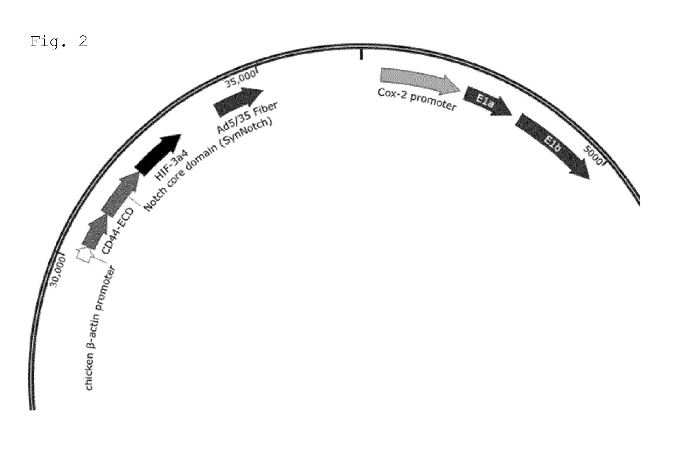

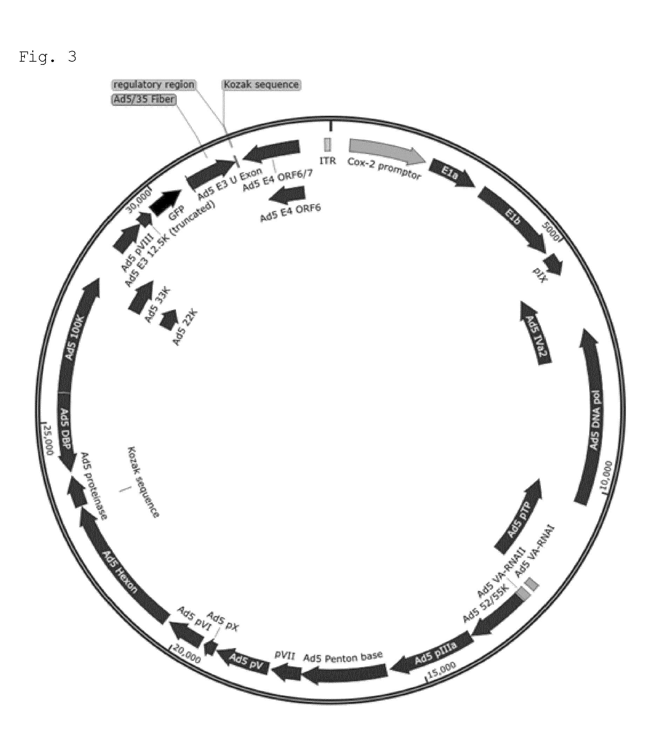

CRAd-synNotch (forward/reverse) and CRAd-GFP are oncolytic, whereas ADX730, wtAd, and Ad-LacZ are not oncolytic. CRAd-synNotch (forward/reverse) and CRAd-GFP have an Ad5/35 chimeric fiber gene containing the knob and shaft portions of a fiber protein derived from Ad35 and the tail portion of a fiber protein derived from Ad5, whereas the other adenoviral vectors do not have the Ad5/35 chimeric fiber gene. Figs. 1 and 2 are schematic diagrams of CRAd-synNotch (forward), and Fig. 3 is a schematic diagram of CRAd-GFP. Fig. 36 shows the base sequence of the Ad5/35 chimeric fiber gene used in this Example (SEQ ID NO: 8), and Fig. 40 shows the amino acid sequence (SEQ ID NO: 14) of the protein encoded by the base sequence of SEQ ID NO: 8.

CRAd-synNotch (forward/reverse), CRAd-GFP, ADX730, wtAd, and Ad-LacZ were subjected to mass culture and purification, and then buffer exchange into an adenovirus preservation solution at a pH of 8.0 containing Tris, sucrose, NaCl, MgCl₂, Tween (trademark) 80, EDTA, and ethanol was performed, using Amicon Ultra-15 (Merck). After aliquoting an appropriate amount of each, they were stored at -80°C until used in subsequent experiments. The composition of the adenovirus preservation solution is shown in the table below.

**Table 12**

| Composition of Adenovirus Preservation Solution | |
|---|---|
| Tris | 5 mM |
| sucrose | 146 mM |
| NaCl | 75 mM |
| MgCl₂ | 1 mM |
| Tween 80 | 0.005% |
| EDTA | 100 *µ*M |
| ethanol | 0.5% |

### 1.4 Cells and Medium

HEK293 cells, human triple-negative breast cancer cell line MDA-MB-231 cells (The European Collection of Cell Cultures), human normal urothelial cell line SV-HUC-1 cells (American Type Culture Collection), and COX-2 protein-negative human breast cancer cell line BT474 cells (American Type Culture Collection) were cultured using D-MEM medium containing 10% FBS, and 1% 100 U/ml penicillin and 100 mg/ml streptomycin (P/S; Nacalai Tesque).

Human bladder cancer cell line T24 cells (American Type Culture Collection) were cultured using Eagle's minimal essential medium (E-MEM; FUJIFILM Wako Pure Chemical Corporation) containing 10% FBS and 1% P/S.

Human prostate cancer cell line DU145 cells (American Type Culture Collection) were cultured using Roswell Park Memorial Institute 1640 (RPMI 1640; FUJIFILM Wako Pure Chemical Corporation) containing 10% FBS and 1% P/S.

Unless otherwise specified, cell culture was performed under conditions of 37°C, 5% CO₂ and 21% O₂.

### 1.5 Analysis of CD46, CAR, and CD44 by Flow Cytometry

The expression levels of CD46, CAR, and CD44 in BT474, T24, MDA-MB-231, and DU145 cells were analyzed using flow cytometry. The specific method is described below.

Each cell line (1 × 10⁶ cells) was washed by centrifugation with PBS. A flow cytometry reaction solution was prepared using FITC ANTI-HUMAN CD46 (BioLegend), PE-Anti-CAR (Merck), and APC ANTI-MOUSE/HUMAN CD44 (BioLegend), added to each cell line, and allowed to react on ice with protection from light for 30 minutes. After the reaction, washing by centrifugation with PBS was performed twice, and analysis was performed using Guava (trademark) easyCyte (trademark) (Merck). As a control, 10 µl of PBS was added to each cell line in place of the flow cytometry reaction solution and allowed to react on ice with protection from light for 30 minutes, and after washing twice with PBS, analysis was performed in the same manner. The obtained data were analyzed using the included software, InCyte.

**Table 13**

| Composition of Flow Cytometry Reaction Solution | |
|---|---|
| APC ANTI-MOUSE/HUMAN CD44 | 1.0*µ*l |
| FITC ANTI-HUMAN CD46 | 5.0*µ*l |
| PE-Anti-CAR | 1.0*µ*l |
| PBS | 3.0*µ*l |

### 1.6 Real-time RT-PCR

### 1.6.1 Expression Analysis of COX-2, HAS-1, HAS-2, and HAS-3 Genes

The expression of COX-2, HAS-1, HAS-2, and HAS-3 genes in T24 cells, MDA-MB-231 cells, and DU145 cells was analyzed by real-time RT-PCR. Regarding COX-2 gene, the relative expression level was analyzed, with the expression level in BT474 cells set as 1. Regarding HAS-1, HAS-2, and HAS-3 genes, the relative expression levels were analyzed, with the expression levels in SV-HUC-1 cells set as 1.

Specifically, T24 cells, MDA-MB-231 cells, DU145 cells, BT474 cells, and SV-HUC-1 cells were seeded at 5 × 10⁵ cells/well in a 6-Well Cell Culture Plate Flat Bottom (Corning) and cultured. The cells were collected the day after seeding, and total RNA was extracted using NucleoSpin (trademark) RNA (TaKaRa). cDNA was synthesized from the extracted total RNA using a PrimeScript (trademark) RT reagent Kit with gDNA Eraser (TaKaRa), and using this as a template, PCR reaction and analysis by the ΔΔCt method were performed in triplicate wells using TB Green (trademark) Premix Ex Taq (trademark) II (TaKaRa). The mRNA levels were normalized by the expression level of the control gene TATA-binding protein (TBP). The primers used for PCR are shown in the tables below.

**Table 14a**

| Real-time PT-PCR Primer List | | |
|---|---|---|
| Primer name | Sequences (5' → 3') | |
| HAS1 Forward | GGAATAACCTCTTGCAGTAGTTTC | SEQ ID NO: 20 |
| HAS1 Reverse | GCCGGTCATCCCCAAAAG | SEQ ID NO: 21 |
| HAS2 Forward | TCGCAACACGTAACGCAAT | SEQ ID NO: 22 |
| HAS2 Reverse | ACTTCTCTTTTTCCACCCCATTT | SEQ ID NO: 23 |
| HAS3 Forward | AACAAGTACGACTCATGGATTTCCT | SEQ ID NO: 24 |
| HAS3 Reverse | GCCCGCTCCACGTTGA | SEQ ID NO: 25 |
| COX-2 Forward | GATACTCAGGCAGAGATGATCTACCC | SEQ ID NO: 26 |
| COX-2 Reverse | AGACCAGGCACCAGACCAAAGA | SEQ ID NO: 27 |
| HIF-3*α*4 Forward | GGGAGACATGGCTTACCTGT | SEQ ID NO: 28 |
| HIF-3*α*4 Reverse | GCGTACTCTTCATGCGCAAG | SEQ ID NO: 29 |
| VEGF Forward | TACCTCCACCATGCCAAGTG | SEQ ID NO: 30 |
| VEGF Reverse | ATGATTCTGCCCTCCTCCTTC | SEQ ID NO: 31 |
| CCL2 Forward | AAGATCTCAGTGCAGAGGCTCG | SEQ ID NO: 32 |
| CCL2 Reverse | TTGCTTGTCCAGGTGGTCCAT | SEQ ID NO: 33 |
| SOX-2 Forward | GACAGTTACGCGCACATGAA | SEQ ID NO: 34 |
| SOX-2 Reverse | TAGGTCTG CGAGCTGGTCAT | SEQ ID NO: 35 |
| TBP Forward | GCCAGCTTCGGAGAGTTCTGGGATT | SEQ ID NO: 36 |
| TBP Reverse | CGGGCACGAAGTGCAATGGTCTTTAA | SEQ ID NO: 37 |

**Table 14b**

| Primer name | Sequences (5' → 3') | |
|---|---|---|
| Cortactin Forward | AAAGCTTCAGCAGGCCAC | SEQ ID NO: 40 |
| Cortactin Reverse | TTTGGTCCTGTTTCAAGTTCC | SEQ ID NO: 41 |
| OCT4 Forward | GGTATTCAGCCAAACGACCA | SEQ ID NO: 42 |
| OCT4 Reverse | CACACTCGGACCACATCCTT | SEQ ID NO: 43 |
| Nanog Forward | GTGATTTGTGGGCCTGAAGA | SEQ ID NO: 44 |
| Nanog Reverse | ACACAGCTGGGTGGAAGAGA | SEQ ID NO: 45 |
| PHD3 Forward | TCCTGCGGATATTTCCAGAGG | SEQ ID NO: 46 |
| PHD3 Reverse | GGTTCCTACGATCTGACCAGAA | SEQ ID NO: 47 |
| GLUT1 Forward | CCAGGGTAGCTGCTGGAGC | SEQ ID NO: 48 |
| GLUT1 Reverse | TGGCATGGCGGGTTGT | SEQ ID NO: 49 |
| CyclinG2 Forward | GCTGAAAGCTTGCAACTGCCGAC | SEQ ID NO: 50 |
| CyclinG2 Reverse | GGTATCGTTGGCAGCTCAGGAAC | SEQ ID NO: 51 |

### 1.6.2 Expression Analysis of HIF-3α4, VEGF, CCL2, and SOX-2 Genes

The expression of HIF-3α4, VEGF, CCL2, and SOX-2 genes in T24 cells and MDA-MB-231 cells was analyzed by real-time RT-PCR. Specifically, each cell line was seeded at 1.0 × 10⁵ cells/well in a 6-Well Cell Culture Plate Flat Bottom (Corning), cultured overnight, and, on the following day, infected with CRAd-synNotch (forward) or ADX730 at an MOI of 50. The cells were cultured under normoxic conditions (21% O₂) or hypoxic conditions (2% O₂) for 48 hours and collected. Thereafter, the expression levels of HIF-3α4, VEGF, CCL2, and SOX-2 genes were analyzed in the same manner as in item 1.6.1, except for the PCR primers. The PCR primers are shown in the tables in item 1.6.1.

### 1.6.3 Expression Analysis of SOX-2, Cortactin, OCT4, Nanog, VEGF, PHD3, GLUT1, and CyclinG2 Genes

The expression of SOX-2, Cortactin, OCT4, Nanog, VEGF, PHD3, GLUT1, and CyclinG2 genes in T24 cells and DU145 cells was analyzed by real-time RT-PCR. Specifically, each cell line was seeded at 5.0 × 10⁵ cells/well in a 6-Well Cell Culture Plate Flat Bottom (Corning) and infected with CRAd-synNotch (forward) or CRAd-GFP at an MOI of 50. Thereafter, under normoxic conditions (21% O₂) or hypoxic conditions (2% O₂), the T24 cells were cultured for 72 hours, and the DU145 cells were cultured for 96 hours. After the cells were collected, the expression levels of SOX-2, Cortactin, OCT4, Nanog, VEGF, PHD3, GLUT1, and CyclinG2 genes were analyzed in the same manner as in item 1.6.1, except for the PCR primers. The PCR primers are shown in the tables in item 1.6.1.

### 1.7 Western Blotting

1.7.1
T24 cells or MDA-MB-231 cells were infected in vitro with CRAd-synNotch (forward) or ADX730, and the increase or decrease in the expression level of the CD44 region in the fusion gene carried thereby was evaluated by Western blotting.

Specifically, each cell line was seeded at 1.0 × 10⁵ cells/well in a 6-well plate (Corning) and cultured overnight. After the culture, the cells were infected with a predetermined amount of adenovirus and cultured for further 48 hours. After the culture, the cells were collected, mixed with sample buffer (for SDS-PAGE, concentrated 6-fold, containing reducing agent) (Nacalai Tesque, Cat. No. 09499-14), heated at 95°C for 5 minutes, cooled on ice, and used as a sample.

Each sample was subjected to SDS-polyacrylamide gel electrophoresis. After the electrophoresis, the gel was blotted onto a polyvinylidene difluoride (PVDF) membrane. The PVDF membrane was washed with a washing solution (PBS containing 0.1% Tween (trademark) 20, hereafter referred to as "PBS-T"), and then blocking was performed using PBS-T containing 5% skim milk (Nacalai Tesque) at room temperature for 1 hour.

After the blocking, the membrane was washed, and a primary antibody reaction was performed with shaking at 4°C overnight, using CD44 (E7K2Y) XP (trademark) Rabbit mAb (Cell Signaling Technology) diluted 1000-fold with Can Get Signal Immunoreaction Enhancer Solution 1 (Toyobo Co., Ltd.) or β-Actin (C4) (Santa Cruz Biotechnology) diluted 1000-fold with PBS-T containing 5% Blocking One (Nacalai Tesque).

After the primary antibody reaction, the membrane was washed, and a secondary antibody reaction was performed with shaking for 1 hour, using Anti-IgG (H+L chain) (Rabbit) p-Ab-HRP (Medical & Biological Laboratories) diluted 1000-fold with Can Get Signal Immunoreaction Enhancer Solution 2 (Toyobo Co., Ltd.) or Anti-IgG (H+L chain) (Mouse) pAb-HRP (Medical & Biological Laboratories) diluted 1000-fold with PBS-T containing 5% Blocking One (Nacalai Tesque). Subsequently, chemiluminescence was detected using Chemi-Lumi One L (Nacalai Tesque).

1.7.2
In addition, T24 cells and DU145 cells were infected with CRAd-synNotch (forward), and the increase or decrease in the expression level of the CD44 region in the fusion gene carried thereby was evaluated by Western blotting. Specifically, the amount of adenovirus used for infection was set to an MOI of 25 or an MOI of 50, and the culture time after infection was set to 24 hours, 48 hours, 72 hours, or 96 hours. For a primary antibody reaction, CD44 (E7K2Y) XP (trademark) Rabbit mAb (Cell Signaling Technology) diluted 1000-fold with Can Get Signal Immunoreaction Enhancer Solution 1 (Toyobo Co., Ltd.), HIF3α4 polyclonal antibody (Hokudo) diluted 1000-fold with Can Get Signal Immunoreaction Enhancer Solution 1, or β-Actin (C4) (Santa Cruz Biotechnology) diluted 1000-fold with PBS-T containing 5% Blocking One (Nacalai Tesque) was used. The other experimental conditions were the same as in item 1.7.1.

### 1.8 Evaluation of Adenoviral Vector Propagation Ability

COX-2-positive T24 and DU145 cells, COX-2-negative BT474 cells, and MDA-MB-231 cells were infected in vitro with wtAd, ADX730, CRAd-GFP, or CRAd-synNotch (forward), and the viral propagation ability driven by the COX-2 promoter and the E1 promoter was evaluated by PCR.

Specifically, each cell line was seeded at 5 × 10⁵ cells/well in a 6-Well Cell Culture Plate Flat Bottom (Corning) and cultured under conditions of 37°C and 5% CO₂ overnight. After the culture, the medium was replaced with 300 µl of medium containing the corresponding viral vector at an MOI of 50, and infection was carried out under the conditions of 37°C and 5% CO₂ for 1 hour. After the infection, 1.7 ml of medium was added, and culture was performed under the conditions of 37°C and 5% CO₂ for 24 hours. After the culture, the cells were collected, and DNA extraction was performed using NucleoSpin (trademark) Tissue (MACHEREY-NAGEL). Using the extracted DNA as a template, quantification and analysis were performed by PCR reaction using TB Green (trademark) Premix Ex Taq (trademark) II (TaKaRa). The primers used for PCR are shown in the table below.

**Table 15**

| PCR Primer List | | |
|---|---|---|
| Primer name | Sequences (5' →3') | |
| Adeno L2 Forward | TTGTGGTTCTTGCAGATATGGC | SEQ ID NO: 38 |
| Adeno L2 Reverse | TCGGAATCCCGGCACC | SEQ ID NO: 39 |

### 1.9 Cancer Cell Proliferation Suppressive Effect of CRAd-synNotch (Forward)

Each of the following cell lines: BT474 cells, T24 cells, MDA-MB-231 cells, and DU145 cells was seeded at 5 × 10³ cells/well in a 96-Well Cell Culture Plate Flat Bottom (Corning) and cultured overnight. Subsequently, the medium was replaced with 100 µl of medium supplemented with ADX730, CRAd-GFP, or CRAd-synNotch (forward) at an MOI of 50, or with fresh medium without recombinant adenovirus (Mock), and culture was performed under the normoxic conditions (normoxia: 21% O₂) or the hypoxic conditions (hypoxia: 2% O₂) for 72 hours. As a colorimetric reagent, 20 µl of 3-(4,5-dimethylthiazol-2-yl)-5-(3-carboxymethoxyphenyl)-2-(4-sulfophenyl)-2H-tetrazolium, inner salt (MTS) (Promega Corporation) was added to the culture supernatant, and, after 4 hours of culture, the absorbance at a wavelength of 492 nm was measured.

### 1.10 Bladder Cancer Treatment Experiment Using Mice

Six-week-old female BALB/c-nu/nu mice were purchased from CLEA Japan and used. 140 µl of a 1:1 mixture of 1 × 10⁶ T24 cells and Matrigel (Becton Dickinson) was inoculated into the right flank of each mouse. Tumor formation was confirmed 13 days after the inoculation, and 20 mice were randomly divided into four groups: a CRAd-synNotch (forward) intratumoral administration group, a CRAd-GFP intratumoral administration group, an ADX730 intratumoral administration group, and a PBS intratumoral administration group (n = 5). CRAd-synNotch (forward), CRAd-GFP, or ADX730 at 1 × 10⁹ infectious units (ifu)/50 µl PBS, or 50 µl of PBS, was intratumorally administered a total of six times at 3-day intervals (Days 0, 4, 8, 12, 16, and 20). The short diameter (W) and long diameter (L) of the tumor were measured, with the start day of treatment designated as Day 0, and the tumor volume was calculated using the formula (W² × L)/2. A tumor volume of 300 mm³ was set as the endpoint.

### 1.11 Immunohistochemical Staining

1 × 10⁶ T24 cells were subcutaneously inoculated into nude mice, and after tumor formation, PBS or 1 × 10⁹ ifu of CRAd-GFP, ADX730, or CRAd-synNotch (forward) was intratumorally administered. The tumor tissue was excised and fixed with paraformaldehyde. Paraffin-embedded T24 tumor tissue sections were deparaffinized and rehydrated. Antigen retrieval was performed using a Bond epitope retrieval buffer (pH 6.0; Leica Microsystems) at 98°C for 20 minutes. Immunohistochemical staining was performed using an automated tissue processor (Leica Microsystems Bond) according to the manufacturer's standard protocol. The tissue sections were incubated with an anti-CD44 antibody (1:600, Catalog#: 37259S, Cell Signaling Technology) at room temperature for 15 minutes. After washing, the sections were incubated with a peroxidase-labelled secondary antibody. Furthermore, after washing, the sections were incubated with 3,3'-diaminobenzidine (Muto Pure Chemicals Co., Ltd.) and counterstained with hematoxylin. The obtained tissue slides were observed using a BZ-X710 fluorescent microscope (Keyence).

### 2. Results

### 2.1 Expression Analysis of CD46, CAR, and CD44 in BT474 Cells, T24 Cells, MDA-MB-231 Cells, and DU145 Cells by Flow Cytometry

### 2.1.1 Expression Analysis of CD46, CAR, and CD44 in BT474 Cells

Flow cytometry was performed on BT474 cells by the method described in item 1.5. Fig. 4 shows the results.

When the flow cytometry reaction solution was used, the mean fluorescence intensity (MFI) of the BT474 cells at the fluorescence wavelength of CD46 was 157.56 ± 2.41. The MFI of a control group using PBS was 17.68 ± 0.09. The significant increase in fluorescence intensity compared with the control group confirmed that CD46 was highly expressed in the BT474 cells.

When the flow cytometry reaction solution was used, the MFI of the BT474 cells at the fluorescence wavelength of CAR was 23.83 ± 0.31. The MFI of the control group was 15.48 ± 0.05. The significant increase in fluorescence intensity compared with the control group confirmed that CAR was highly expressed in the BT474 cells.

When the flow cytometry reaction solution was used, the MFI of the BT474 cells at the fluorescence wavelength of CD44 was 22.67 ± 0.09. The MFI of the control group was 30.54 ± 0.05. Since there was no significant difference in fluorescence intensity compared with the control group, it was confirmed that CD44 was not highly expressed in the BT474 cells.

### 2.1.2 Expression Analysis of CD46, CAR, and D44 in T24 Cells

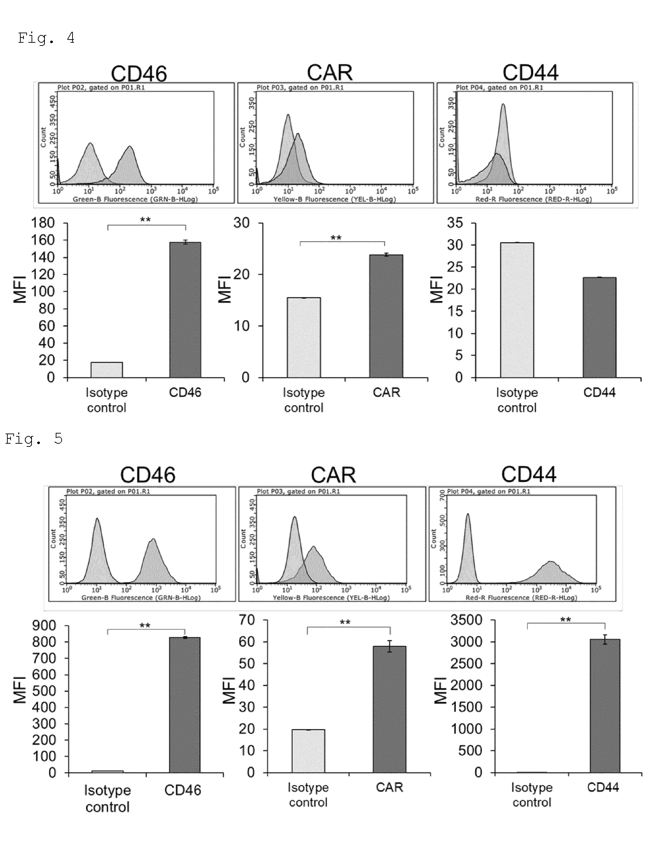

Flow cytometry was performed on T24 cells by the method described in item 1.5. Fig. 5 shows the results.

When the flow cytometry reaction solution was used, the MFI of the T24 cells at the fluorescence wavelength of CD46 was 826.67 ± 5.50. The MFI of a control group using PBS was 11.59 ± 0.07. The significant increase in fluorescence intensity compared with the control group confirmed that CD46 was highly expressed in the T24 cells.

When the flow cytometry reaction solution was used, the MFI of the T24 cells at the fluorescence wavelength of CAR was 57.90 ± 2.63. The MFI of the control group was 19.67 ± 0.09. The significant increase in fluorescence intensity compared with the control group confirmed that CAR was highly expressed in the T24 cells.

When the flow cytometry reaction solution was used, the MFI of the T24 cells at the fluorescence wavelength of CD44 was 3058.09 ± 107.04. The MFI of the control group was 4.97 ± 0.01. The significant increase in fluorescence intensity compared with the control group confirmed that CD44 was highly expressed in the T24 cells.

### 2.1.3 Expression Analysis of CD46, CAR, and CD44 in MDA-MB-231 Cells

Flow cytometry was performed on MDA-MB-231 cells by the method described in item 1.5. Fig. 6 shows the results.

When the flow cytometry reaction solution was used, the MFI of the MDA-MB-231 cells at the fluorescence wavelength of CD46 was 838.49 ± 10.80. The MFI of a control group using PBS was 38.60 ± 0.25. The significant increase in fluorescence intensity compared with the control group confirmed that CD46 was highly expressed in the MDA-MB-231 cells.

When the flow cytometry reaction solution was used, the MFI of the MDA-MB-231 cells at the fluorescence wavelength of CAR was 33.36 ± 0.49. The MFI of the control group was 67.55 ± 0.39. Since there was no significant difference in fluorescence intensity compared with the control group, it was confirmed that CAR was not highly expressed in the MDA-MB-231 cells.

When the flow cytometry reaction solution was used, the MFI of the MDA-MB-231 cells at the fluorescence wavelength of CD44 was 5869.71 ± 32.15. The MFI of the control group was 5.62 ± 0.02. The significant increase in fluorescence intensity compared with the control group confirmed that CD44 was highly expressed in the MDA-MB-231 cells.

### 2.1.4 Expression Analysis of CD46, CAR, and CD44 in DU145 Cells

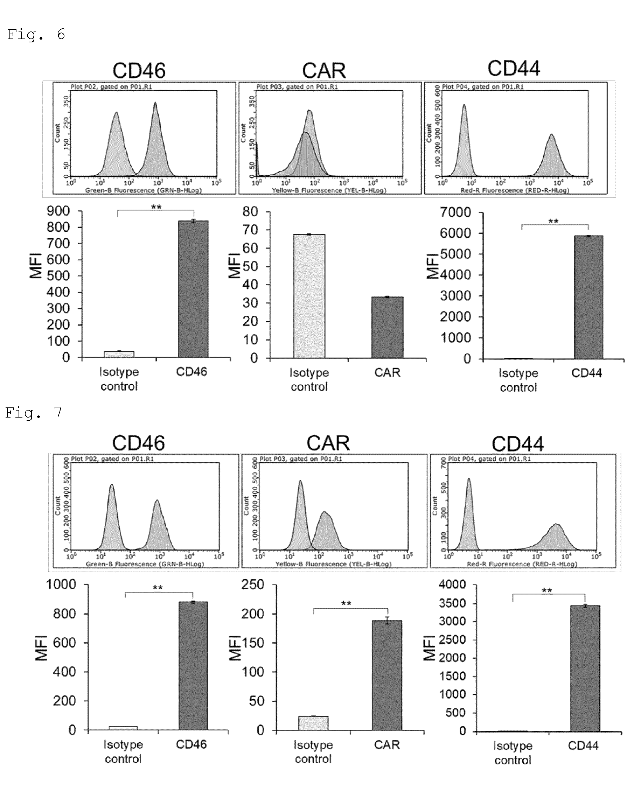

Flow cytometry was performed on DU145 cells by the method described in item 1.5. Fig. 7 shows the results.

When the flow cytometry reaction solution was used, the MFI of the DU145 cells at the fluorescence wavelength of CD46 was 881.86 ± 6.87. The MFI of the control group using PBS was 24.16 ± 0.62. The significant increase in fluorescence intensity compared with the control group confirmed that CD46 was highly expressed in the DU145 cells.

When the flow cytometry reaction solution was used, the MFI of the DU145 cells at the fluorescence wavelength of CAR was 188.65 ± 6.18. The MFI of the control group was 23.97 ± 0.45. The significant increase in fluorescence intensity compared with the control group confirmed that CAR was highly expressed in the DU145 cells.

When the flow cytometry reaction solution was used, the MFI of the DU145 cells at the fluorescence wavelength of CD44 was 3434.24 ± 38.66. The MFI of the control group was 4.79 ± 0.04. The significant increase in fluorescence intensity compared with the control group confirmed that CD44 was highly expressed in the DU145 cells.

### 2.2 Expression Quantification of COX-2 Gene in T24 Cells, MDA-MB-231 Cells, and DU145 Cells

Real-time PT-PCR was performed by the method described in item 1.6.1, and the expression levels of COX-2 gene in T24 cells, MDA-MB-231 cells, and DU145 cells were evaluated by comparing them with the expression level of COX-2 gene in BT474 cells, a human breast cancer cell line that is negative for COX-2 protein. Fig. 8 shows the results.

When the expression level of COX-2 gene in the BT474 cells was set to 1, the relative expression levels of COX-2 gene in the T24 cells, MDA-MB-231 cells, and DU145 cells were all significantly higher. These results confirmed that COX-2 gene was highly expressed in the T24 cells, MDA-MB-231 cells, and DU145 cells.

### 2.3 Expression Quantification of HAS-1, HAS-2, and HAS-3 Genes in T24 Cells, MDA-MB-231 Cells, and DU145 Cells

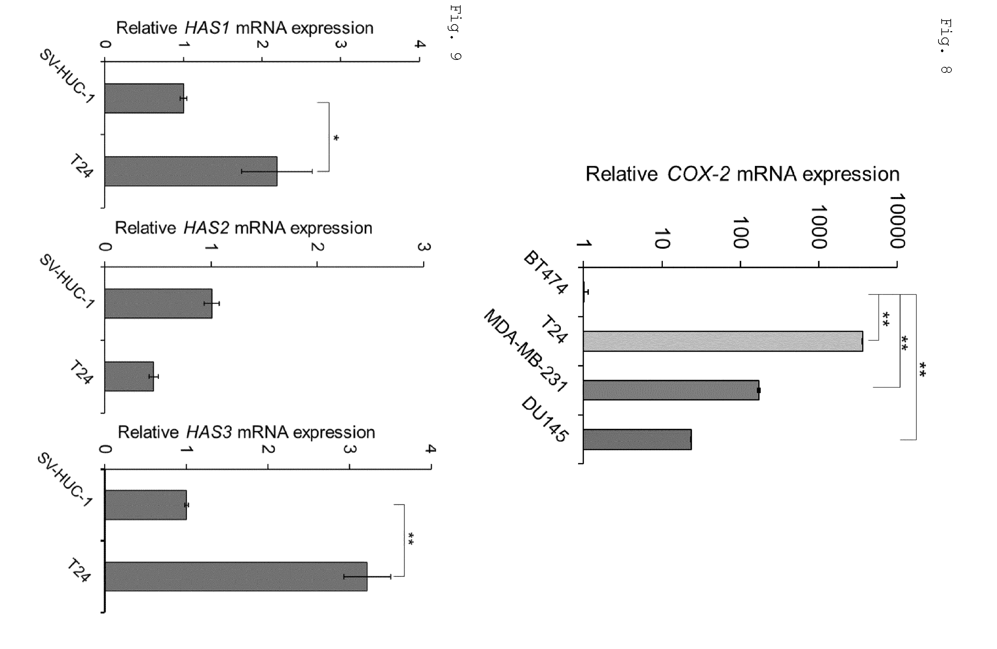

Real-time PT-PCR was performed by the method described in item 1.6.1, and the expression levels of HAS-1, HAS-2, and HAS-3 genes (in other words, hyaluronic acid production ability) in T24 cells, MDA-MB-231 cells, and DU145 cells were evaluated by comparing them with those in SV-HUC-1 cells, a human normal urothelial cell line. Figs. 9-11 show the results.

### 2.3.1 T24 Cells

When the expression levels in the SV-HUC-1 cells were set to 1, the relative expression levels of hyaluronic acid production genes HAS-1 and HAS-3 in the T24 cells were significantly higher. On the other hand, no significant difference was observed in the relative expression level of HAS-2 (Fig. 9).

### 2.3.2 MDA-MB-231 Cells

When the expression levels in the SV-HUC-1 cells were set to 1, the relative expression level of hyaluronic acid production gene HAS-2 in the MDA-MB-231 cells was significantly higher. On the other hand, no significant difference was observed in the relative expression levels of HAS-1 and HAS-3 (Fig. 10).

### 2.3.3 DU145 Cells

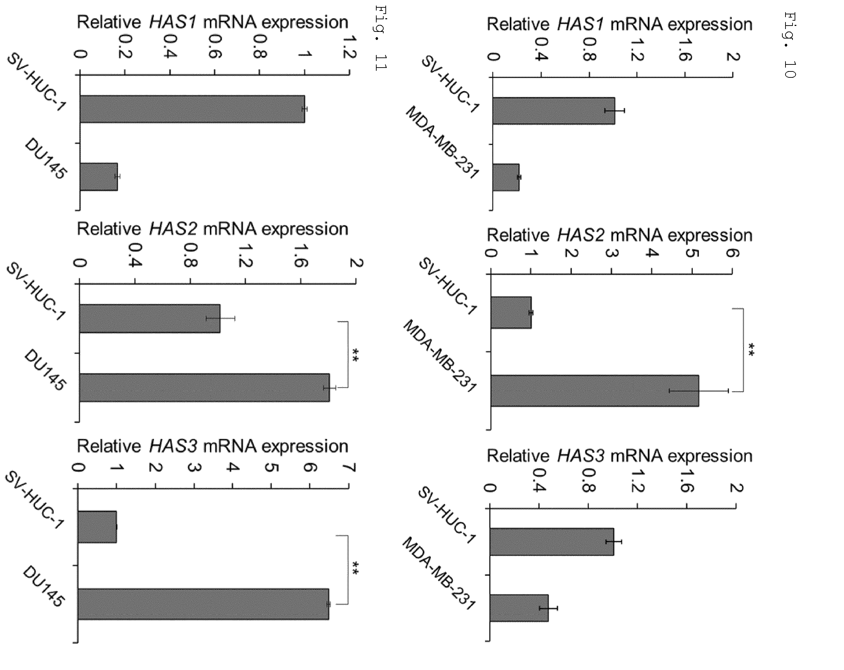

When the expression levels in the SV-HUC-1 cells were set to 1, the relative expression levels of hyaluronic acid production genes HAS-2 and HAS-3 in the DU145 cells were significantly higher. On the other hand, no significant difference was observed in the relative expression level of HAS-1 (Fig. 11).

### 2.4 Titer Measurement of Recombinant Adenoviral Vectors

The titer of each constructed adenoviral vector was measured by the method described in item 1.1.9. The titer of CRAd-synNotch (reverse) was 1.7 × 10¹⁰ ifu/ml. The titer of CRAd-synNotch (forward) was 1.2 × 10¹² ifu/ml. The titer of CRAd-GFP was 1.05 × 10¹¹ ifu/ml.

### 2.5 Expression Analysis of CD44/Notch/HIF-3α4 Fusion Gene in MDA-MB-231 Cells Infected with CRAd-synNotch (Reverse)

The expression of the CD44/Notch/HIF-3α4 fusion gene in MDA-MB-231 cells infected with CRAd-synNotch (reverse) constructed in item 1.1 was evaluated by Western blotting and real-time RT-PCR. CRAd-synNotch (reverse) and other adenoviral vectors were those obtained by performing mass culture from tertiary virus solutions and purification by the method described in item 1.1.8. The specific results are shown below.

### 2.5.1 Expression Analysis of CD44 by Western Blotting

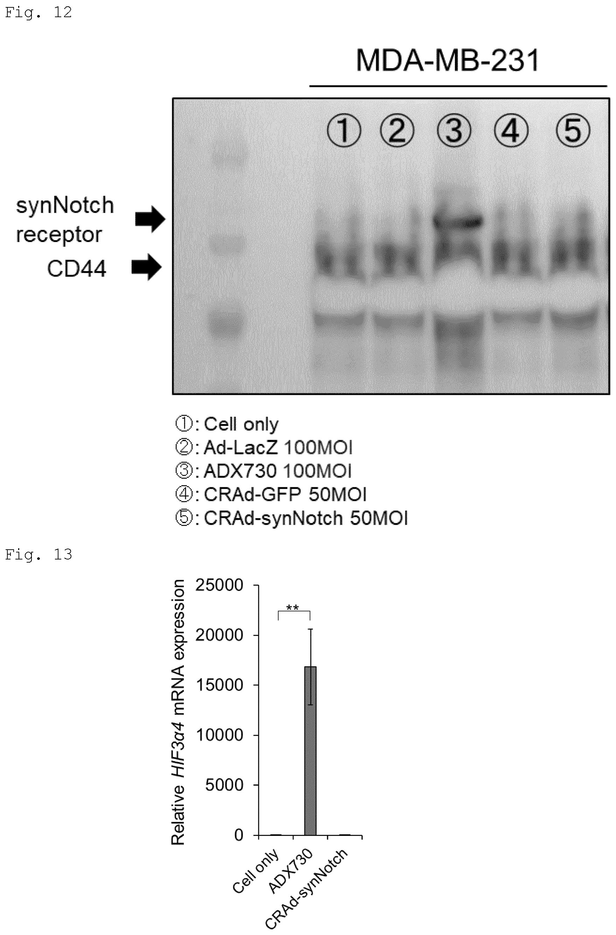

Western blotting was performed on MDA-MB-231 cells not infected with adenovirus (Cell only), MDA-MB-231 cells infected with Ad-LacZ at an MOI (Multiplicity of Infection) of 100, MDA-MB-231 cells infected with ADX730 at an MOI of 100, MDA-MB-231 cells infected with CRAd-GFP at an MOI of 50, and MDA-MB-231 cells infected with CRAd-synNotch (reverse) at an MOI of 50, by the method described in item 1.7.1. Fig. 12 shows the results.

In all of the samples, a band (approximately 80 kDa) assigned to endogenous CD44 was observed. On the other hand, a band (approximately 105 kDa) considered to be the CD44/Notch/HIF-3α4 fusion protein was observed only in the ADX730-infected cells, and was not observed in the CRAd-synNotch (reverse)-infected cells.

### 2.5.2 Expression Quantification of HIF-3α4 Gene by Real-Time RT-PCR

Real-time RT-PCR was performed on MDA-MB-231 cells not infected with adenovirus (Cell only), MDA-MB-231 cells infected with ADX730 at an MOI of 100, and MDA-MB-231 cells infected with CRAd-synNotch (reverse) at an MOI of 50, by the method described in item 1.6.2, and the expression levels of HIF-3α4 gene were quantified. Fig. 13 shows the results.

The expression level of HIF-3α4 gene in the MDA-MB-231 cells infected with ADX730 was significantly higher than that in the MDA-MB-231 cells not infected with adenovirus. On the other hand, the expression level of HIF-3α4 gene in the MDA-MB-231 cells infected with CRAd-synNotch (reverse) showed no significant difference compared with the MDA-MB-231 cells not infected with adenovirus.

### 2.5.3. Summary

In general, in the construction of adenoviral vectors, when it is intended to express two sets of genes (in this test example, the CD44/Notch/HIF-3α4 fusion gene, and E1A/E1B genes) under the control of different promoters (in this test example, the β-actin promoter and the COX-2 promoter), it is common practice to arrange the two sets of genes in reverse orientations. Thus, the present inventors first investigated CRAd-synNotch (reverse), in which the CD44/Notch/HIF-3α4 fusion gene and the E1A/E1B genes are arranged in reverse orientations. However, as described above, no expression of the CD44/Notch/HIF-3α4 fusion gene was observed from CRAd-synNotch (reverse).

Thus, the present inventors investigated CRAd-synNotch (forward), in which the CD44/Notch/HIF-3α4 fusion gene and the E1A/E1B genes were arranged in the same orientation (forward orientation). The results are shown in item 2.6 and subsequent items.

### 2.6 Expression Analysis of CD44/Notch/HIF-3α4 Fusion Gene in MDA-MB-231 Cells Infected with CRAd-synNotch (Forward)

The expression of the CD44/Notch/HIF-3α4 fusion gene in MDA-MB-231 cells infected with CRAd-synNotch (forward) was evaluated by Western blotting and RT-PCR, as in item 2.5. For CRAd-synNotch (forward), a secondary virus solution was used. The specific results are shown below.

### 2.6.1 Expression Analysis of CD44 by Western Blotting

### 2.6.1.1 CRAd-synNotch (Forward) Clone 1

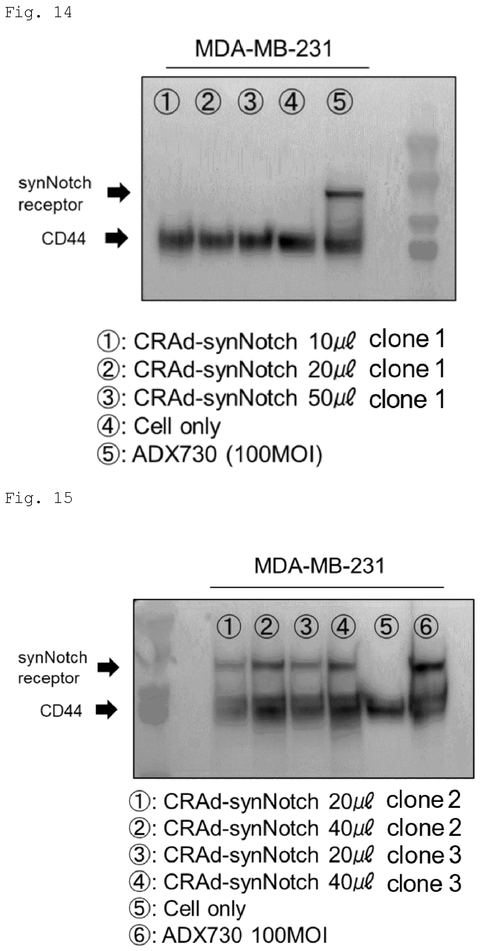

Western blotting was performed by the method described in item 1.7.1 on MDA-MB-231 cells infected with 10 µl, 20 µl, or 50 µl of a secondary virus solution of CRAd-synNotch (forward) clone 1, MDA-MB-231 cells not infected with adenovirus (Cell only), and MDA-MB-231 cells infected with ADX730 at an MOI of 100. Fig. 14 shows the results.

In all of the samples, a band (approximately 80 kDa) assigned to endogenous CD44 was observed. On the other hand, a band (approximately 105 kDa) considered to be the CD44/Notch/HIF-3α4 fusion protein was observed only in the ADX730-infected cells, and was not observed in the CRAd-synNotch (forward) clone 1-infected cells.

### 2.6.1.2 CRAd-synNotch (Forward) Clone 2 and Clone 3

Western blotting was performed by the method described in item 1.7.1 on MDA-MB-231 cells infected with 20 µl or 40 µl of a secondary virus solution of CRAd-synNotch (forward) clone 2, MDA-MB-231 cells infected with 20 µl or 40 µl of a secondary virus solution of CRAd-synNotch (forward) clone 3, MDA-MB-231 cells not infected with adenovirus (Cell only), and MDA-MB-231 cells infected with ADX730 at an MOI of 100. Fig. 15 shows the results.

In all of the samples, a band (approximately 80 kDa) assigned to endogenous CD44 was observed. Furthermore, a band (approximately 105 kDa) considered to be the CD44/Notch/HIF-3α4 fusion protein was observed in the cells infected with CRAd-synNotch (forward) clone 2, the cells infected with CRAd-synNotch (forward) clone 3, and the ADX730-infected cells.

### 2.6.2 Expression Quantification of CD44 Gene and HIF-3α4 Gene by Real-Time RT-PCR

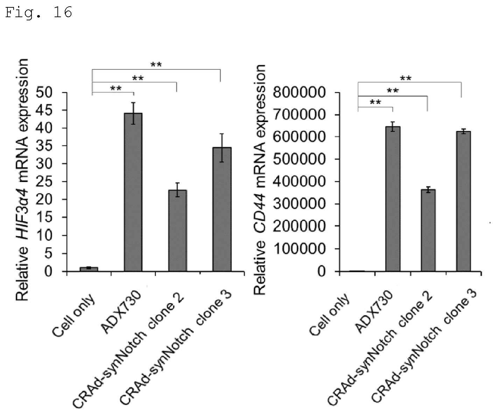

Real-time RT-PCR was performed by the method described in item 1.6.2 on MDA-MB-231 cells not infected with adenovirus (Cell only), MDA-MB-231 cells infected with ADX730 at an MOI of 100, MDA-MB-231 cells infected with 20 µl of CRAd-synNotch (forward) clone 2, and MDA-MB-231 cells infected with 20 µl of CRAd-synNotch (forward) clone 3, and the expression levels of HIF-3α4 gene and CD44 gene were quantified. Fig. 16 shows the results.

In all of the MDA-MB-231 cells infected with ADX730, the MDA-MB-231 cells infected with CRAd-synNotch (forward) clone 2, and the MDA-MB-231 cells infected with CRAd-synNotch (forward) clone 3, the expression levels of HIF-3α4 gene and CD44 gene were significantly higher than those in the MDA-MB-231 cells not infected with adenovirus.

Since the expression levels of HIF-3α4 gene and CD44 gene were particularly high in the MDA-MB-231 cells infected with CRAd-synNotch (forward) clone 3, the present inventors used CRAd-synNotch (forward) clone 3 in subsequent tests, and mass culture and purification of CRAd-synNotch (forward) clone 3 were performed by the method described in item 1.1.8.

### 2.7 Expression Analysis of CD44/Notch/HIF-3α4 Fusion Gene in MDA-MB-231 Cells and T24 Cells Infected with CRAd-synNotch (Forward) Clone 3 after Mass Culture

In MDA-MB-231 cells and T24 cells infected with CRAd-synNotch (forward) clone 3 that had been mass-cultured and purified by the method described in item 1.1.8, the expression of the CD44/Notch/HIF-3α4 fusion gene was evaluated by Western blotting and real-time RT-PCR. Specifically, the results are shown below. In subsequent test examples, CRAd-synNotch (forward) clone 3 is simply referred to as "CRAd-synNotch."

### 2.7.1 Expression Analysis of CD44 by Western Blotting

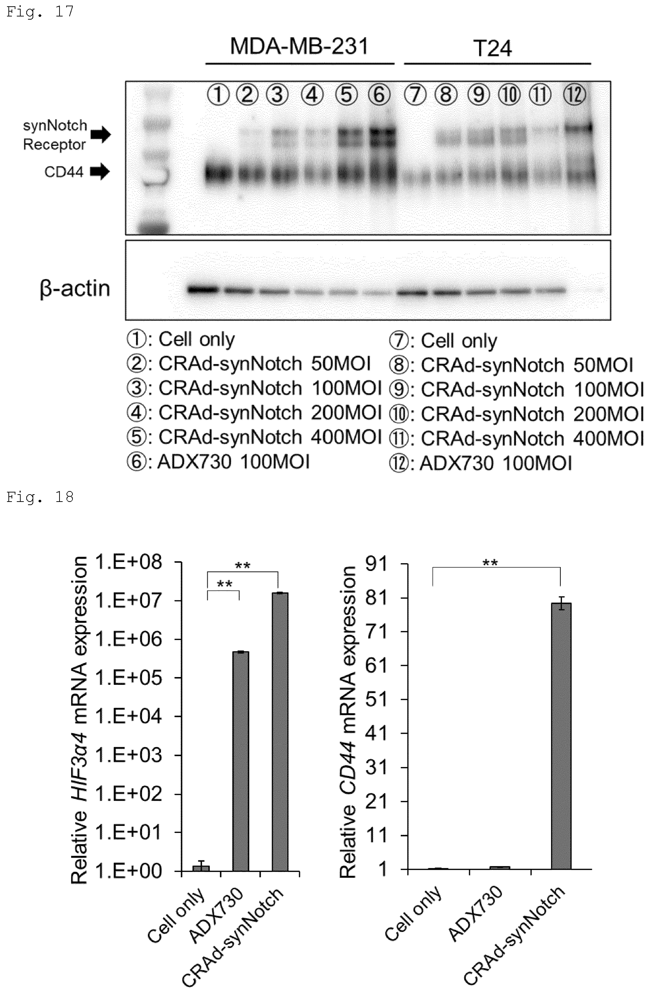

Western blotting was performed by the method described in item 1.7.1 on MDA-MB-231 cells not infected with adenovirus (Cell only), MDA-MB-231 cells infected with CRAd-synNotch at an MOI of 50, 100, 200, or 400, and MDA-MB-231 cells infected with ADX730 at an MOI of 100. In addition, Western blotting was performed by the method described in item 1.7.1 on T24 cells not infected with adenovirus (Cell only), T24 cells infected with CRAd-synNotch at an MOI of 50, 100, 200, or 400, and T24 cells infected with ADX730 at an MOI of 100. Fig. 17 shows the results.

In all of the samples, a band (approximately 80 kDa) assigned to endogenous CD44 was observed. Furthermore, a band (approximately 105 kDa) considered to be the CD44/Notch/HIF-3α4 fusion protein was observed in the MDA-MB-231 cells and T24 cells infected with CRAd-synNotch and the MDA-MB-231 cells and T24 cells infected with ADX730.

### 2.7.2 Expression Quantification of CD44 Gene and HIF-3α4 Gene by Real-Time RT-PCR

Real-time RT-PCR was performed by the method described in item 1.6.2 on T24 cells not infected with adenovirus (Cell only), T24 cells infected with ADX730 at an MOI of 100, and T24 cells infected with CRAd-synNotch at an MOI of 50, and the expression levels of HIF-3α4 gene and CD44 gene were quantified. Fig. 18 shows the results.

The expression levels of HIF-3α4 gene in both the T24 cells infected with ADX730 and the T24 cells infected with CRAd-synNotch were significantly higher than that in the T24 cells not infected with adenovirus.

The expression level of CD44 gene in the T24 cells infected with ADX730 was not significantly different from that in the T24 cells not infected with adenovirus. On the other hand, the expression level of CD44 gene in the T24 cells infected with CRAd-synNotch was significantly higher than that in the T24 cells not infected with adenovirus.

### 2.8 Evaluation of Propagation Ability of Adenoviral Vectors

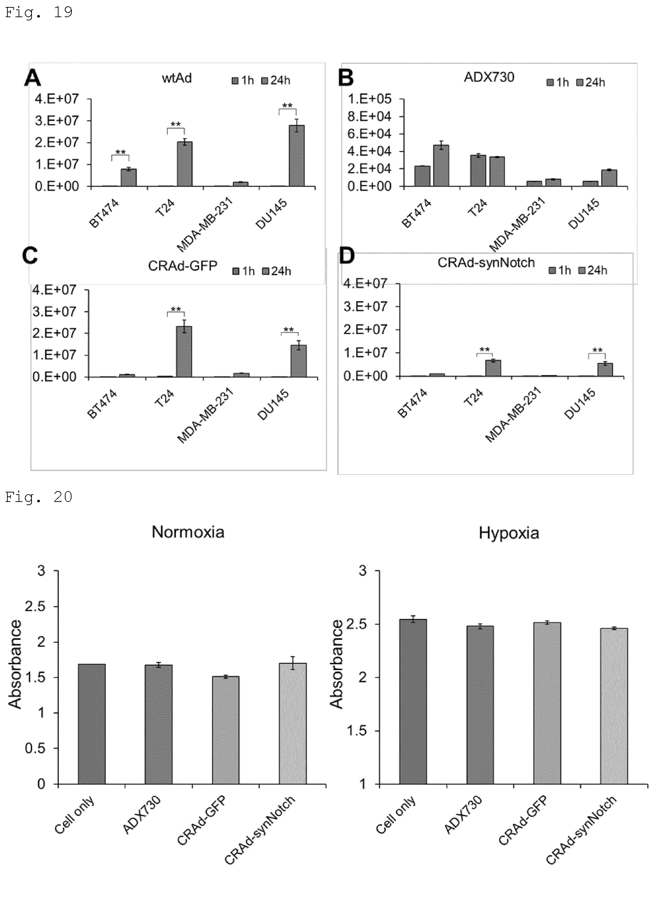

The propagation ability of wtAd, ADX730, CRAd-GFP, and CRAd-synNotch in BT474 cells, T24 cells, MDA-MB-231 cells, and DU145 cells was evaluated by the method described in item 1.8. Fig. 19 shows the results.

### 2.8.1 wtAd

The copy number of wtAd in each of the infected BT474, T24, and DU145 cell lines significantly increased at 24 hours after infection compared with at 1 hour after infection. On the other hand, the copy number of wtAd in the infected MDA-MB-231 cells showed no significant difference at 24 hours after infection compared with at 1 hour after infection (Fig. 19A).

### 2.8.2 ADX730

The copy number of ADX730 in each of the infected BT474, T24, MDA-MB-231, and DU145 cancer cell lines showed no significant difference at 24 hours after infection compared with at 1 hour after infection (Fig. 19B).

### 2.8.3 CRAd-GFP and CRAd-synNotch

The copy numbers of CRAd-GFP and CRAd-synNotch in the infected COX-2-positive T24 and DU145 cells significantly increased at 24 hours after infection than at 1 hour after infection. On the other hand, the copy numbers of CRAd-GFP and CRAd-synNotch in the infected COX-2-negative BT474 cells and the infected MDA-MB-231 cells showed no significant difference at 24 hours after infection compared with at 1 hour after infection (Fig. 19C and Fig. 19D).

### 2.9 Evaluation of Cancer Cell Proliferation Suppressive Effect of CRAd-synNotch

The cell proliferation suppressive effect of CRAd-synNotch on BT474 cells, T24 cells, MDA-MB-231 cells, and DU145 cells was evaluated in vitro by the method described in item 1.9. Figs. 20 to 23 show the results. Note that "Cell only" refers to the case in which, as a mock treatment, medium without adenovirus was added in place of the recombinant adenovirus-containing medium.

### 2.9.1 BT474 Cells

No significant cell proliferation suppressive effect of each of ADX730, CRAd-GFP, and CRAd-synNotch was observed on BT474 cells in normoxic conditions (normoxia: 21% O₂) or hypoxic conditions (hypoxia: 2% O₂), compared with the "Cell only" group (Fig. 20).

### 2.9.2 T24 Cells

A significant cell proliferation suppressive effect of each of ADX730, CRAd-GFP, and CRAd-synNotch was observed on T24 cells in normoxic conditions and hypoxic conditions, compared with the "Cell only" group. In particular, CRAd-synNotch showed a significantly higher cell proliferation suppressive effect than those of ADX730 and CRAd-GFP (Fig. 21).

### 2.9.3 MDA-MB-231 Cells

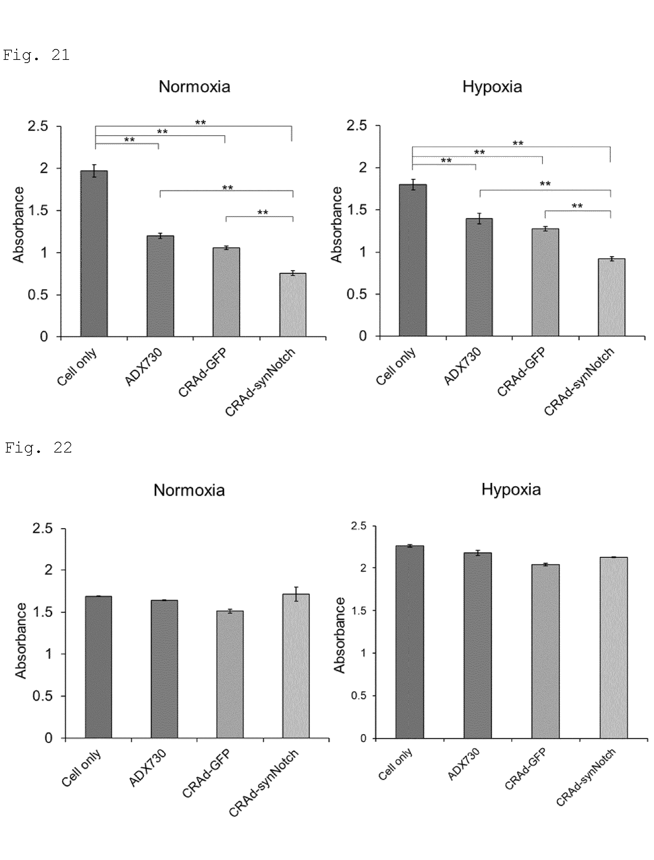

No significant cell proliferation suppressive effect of each of ADX730, CRAd-GFP, and CRAd-synNotch was observed on MDA-MB-231 cells in normoxic conditions or hypoxic conditions, compared with the "Cell only" group (Fig. 22).

### 2.9.4 DU145 Cells

Under normoxic conditions, only CRAd-synNotch showed a significant cell proliferation suppressive effect on DU145 cells compared with the "Cell only" group, whereas neither ADX730 nor CRAd-GFP showed a significant cell proliferation suppressive effect compared with the "Cell only" group. Under hypoxic conditions, ADX730 and CRAd-synNotch showed a significant cell proliferation suppressive effect on DU145 cells compared with the "Cell only" group, whereas CRAd-GFP did not show a significant cell proliferation suppressive effect compared with the "Cell only" group (Fig. 23).

### 2.10 Evaluation of Suppressive Effect of CRAd-synNotch on Gene Downstream of HIF-1α and Genes Downstream of CD44

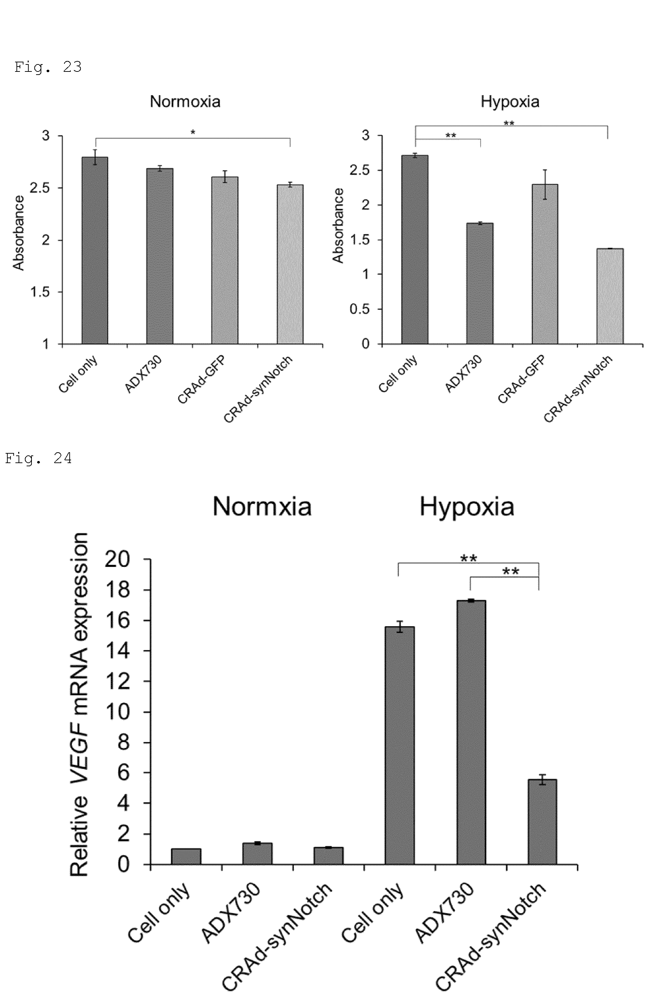

Using the method described in item 1.6.2, the expression of VEGF, which is a gene downstream of HIF-1α, and SOX-2 and CCL2, which are genes downstream of CD44, was analyzed in T24 cells not infected with adenovirus (Cell only), T24 cells infected with ADX730 at an MOI of 50, and T24 cells infected with CRAd-synNotch at an MOI of 50. Figs. 24 to 26 show the results.

### 2.10.1 VEGF Gene

Under normoxic conditions, the expression level of VEGF, which is a gene downstream of HIF-1α, was low in all of the following: T24 cells not infected with adenovirus (Cell only), T24 cells infected with ADX730, and T24 cells infected with CRAd-synNotch. On the other hand, under hypoxic conditions, high VEGF expression was observed in the T24 cells not infected with adenovirus (Cell only) and the T24 cells infected with ADX730; however, the expression level of VEGF in the T24 cells infected with CRAd-synNotch was significantly lower than those in the T24 cells not infected with adenovirus (Cell only) and the T24 cells infected with ADX730 (Fig. 24).

### 2.10.2 SOX-2 Gene

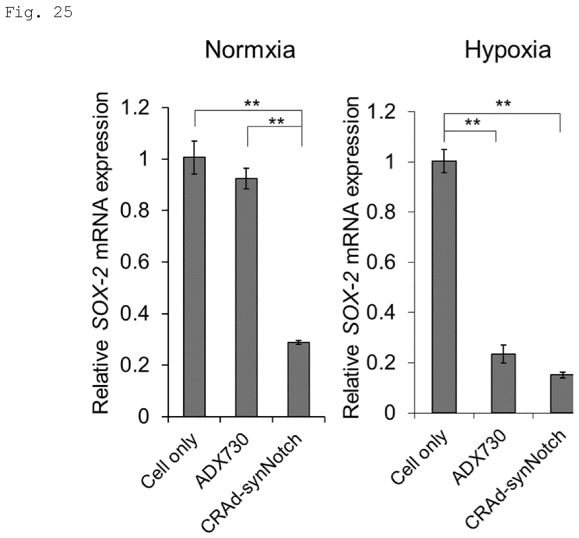

Under normoxic conditions, the expression level of SOX-2, which is a gene downstream of CD44, in the T24 cells infected with CRAd-synNotch was significantly lower than those in the T24 cells not infected with adenovirus (Cell only) and the T24 cells infected with ADX730. Under hypoxic conditions, the expression levels of SOX-2, which is a gene downstream of CD44, in the T24 cells infected with ADX730 and the T24 cells infected with CRAd-synNotch were significantly lower than that in the T24 cells not infected with adenovirus (Cell only) (Fig. 25).

### 2.10.3 CCL2 Gene

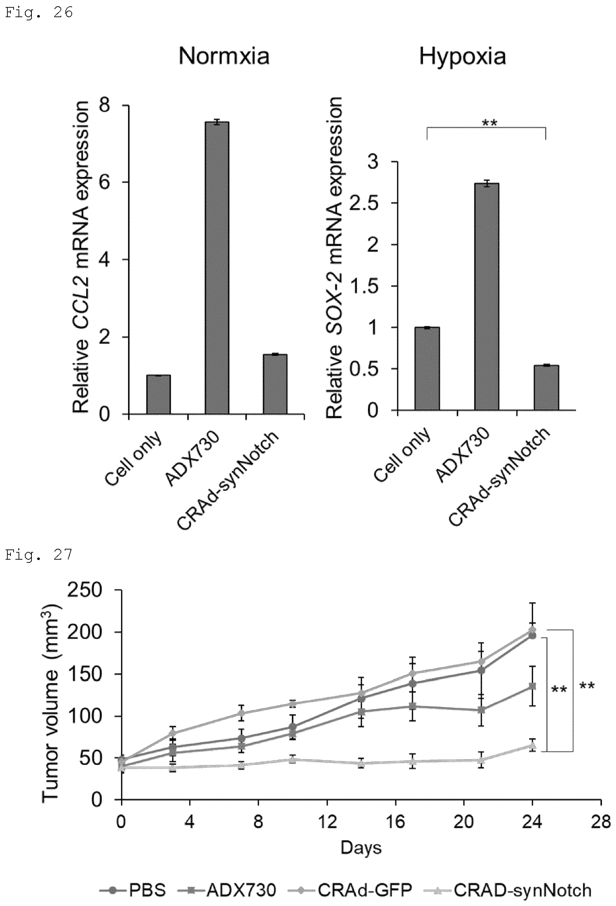

Under normoxic conditions, the expression level of CCL2, which is a gene downstream of CD44, in the T24 cells infected with CRAd-synNotch showed no significant difference compared with the T24 cells not infected with adenovirus (Cell only). On the other hand, under hypoxic conditions, the expression level of CCL2, which is a gene downstream of CD44, in the T24 cells infected with CRAd-synNotch was significantly lower than that in the T24 cells not infected with adenovirus (Cell only) (Fig. 26).

### 2.10.4 Summary

These results suggest that the decoy function of the CD44 extracellular part contained in CRAd-synNotch suppresses the expression of the genes downstream of CD44, and that the HIF-3α4 part contained in CRAd-synNotch suppresses the expression of the gene downstream of HIF-1α.

### 2.11 Evaluation of Bladder Cancer Treatment Effect of CRAd-synNotch In Vivo

Using the method described in item 1.10, PBS, ADX730, CRAd-GFP, or CRAd-synNotch was intratumorally administered to nude mice implanted with T24 cells, and the mice were monitored. Figs. 27 and 28 show the results.

At 24 days after the start of administration of PBS, ADX730, CRAd-GFP, or CRAd-synNotch (Day 24), tumor growth was significantly suppressed in the CRAd-synNotch administration group compared with the PBS administration group and the CRAd-GFP administration group. In addition, the CRAd-synNotch administration group tended to have a higher tumor growth suppressive effect than the ADX730 administration group (Fig. 27).

After completing a total of six administrations, continued observation revealed that survival was significantly prolonged in the CRAd-synNotch administration group compared with the PBS administration group and the CRAd-GFP administration group. In addition, the CRAd-synNotch administration group tended to show longer survival than the ADX730 administration group (Fig. 28).

### 2.12 Expression Analysis of Fusion Protein by Western Blotting

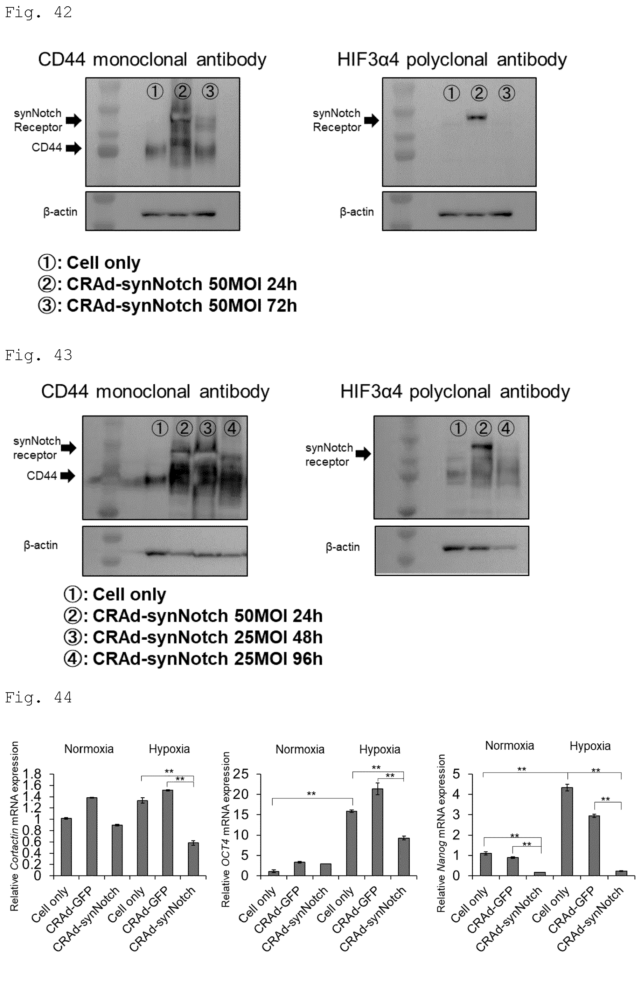

Using the method described in item 1.7.2, T24 cells were infected with CRAd-synNotch at an MOI of 50 and cultured for 24 hours or 72 hours. After the culture, Western blotting was performed. The results of Western blotting using a CD44 antibody as a primary antibody are shown on the left side of Fig. 42. The results of Western blotting using an HIF-3α4 antibody as a primary antibody are shown on the right side of Fig. 42. As shown on the left side of Fig. 42, an endogenous CD44 band (approximately 80 kDa) was observed in all samples. In addition, in the CRAd-synNotch-infected cells, a band (approximately 105 kDa) considered to be the CD44-ECD/Notch/HIF-3α4 fusion protein was observed in both the cells cultured for 24 hours after infection and the cells cultured for 72 hours after infection. The band was observed on the lower molecular weight side in the cells cultured for 72 hours after infection. This result suggests that HIF-3α4 was cleaved from the fusion protein. As shown on the right side of Fig. 42, when the HIF-3α4 antibody was used as the primary antibody, a band (approximately 105 kDa) considered to be the CD44-ECD/Notch/HIF-3α4 fusion protein was observed in the CRAd-synNotch-infected cell cultured for 24 hours after infection, but this band was not observed in the CRAd-synNotch-infected cells cultured for 72 hours after infection. This result also suggests that HIF-3α4 was cleaved from the fusion protein.

Using the method described in item 1.7.2, DU145 cells were infected with CRAd-synNotch at an MOI of 25 or an MOI of 50 and cultured for 24 hours, 48 hours, or 96 hours. After the culture, Western blotting was performed. The results of Western blotting using a CD44 antibody as a primary antibody are shown on the left side of Fig. 43. The results of Western blotting using an HIF-3α4 antibody as a primary antibody are shown on the right side of Fig. 43. As shown on the left side of Fig. 43, an endogenous CD44 band (approximately 80 kDa) was observed in all samples. In addition, in the CRAd-synNotch-infected cells, a band (approximately 105 kDa) considered to be the CD44-ECD/Notch/HIF-3α4 fusion protein was observed in all of the cells cultured for 24 hours after infection, the cells cultured for 48 hours after infection, and the cells cultured for 96 hours after infection. The band was observed on the lower molecular weight side in the cells cultured for 96 hours after infection. This result suggests that HIF-3α4 was cleaved from the fusion protein. As shown on the right side of Fig. 43, when the HIF-3α4 antibody was used as the primary antibody, a band (approximately 105 kDa) considered to be the CD44-ECD/Notch/HIF-3α4 fusion protein was observed in the CRAd-synNotch-infected cells cultured for 24 hours after infection, but this band was not observed in the CRAd-synNotch-infected cells cultured for 96 hours after infection. This result also suggests that HIF-3α4 was cleaved from the fusion protein.

### 2.13 Suppression of Genes Downstream of CD44 and Genes Downstream of HIF-1α by CRAd-synNotch

As described in item 1.6.3, T24 cells were infected with CRAd-GFP at an MOI of 50 or CRAd-synNotch at an MOI of 50, and DU145 cells were infected with CRAd-GFP at an MOI of 25 or CRAd-synNotch at an MOI of 25. The cells were then cultured under normoxic conditions (normoxia: 21% O₂) or hypoxic conditions (hypoxia: 2% O₂) for 72 hours or 96 hours. After the culture, the expression levels of genes downstream of CD44 (Cortactin, OCT4, Nanog, and SOX-2) and the expression levels of genes downstream of HIF-1α (VEGF, GLUT1, CyclinG2, and PHD3) were analyzed by real-time RT-PCR. Figs. 44 to 47 show the results.

Fig. 44 shows the results of analyzing the expression levels of Cortactin (Fig. 44 (left)), OCT4 (Fig. 44 (center)), and Nanog (Fig. 44 (right)), which are genes downstream of CD44, in the T24 cells. As shown in Fig. 44, under hypoxic conditions, the expression levels of Cortactin, OCT4, and Nanog in the CRAd-synNotch-infected cells were significantly lower than those in the uninfected cells and CRAd-GFP-infected cells.

Fig. 45 shows the results of analyzing the expression levels of PHD3 (Fig. 45 (left)), GLUT1 (Fig. 45 (center)), and CyclinG2 (Fig. 45 (right)), which are genes downstream of HIF-1α, in the T24 cells. As shown in Fig. 45, under hypoxic conditions, the expression levels of PHD3, GLUT1, and CyclinG2 in the CRAd-synNotch-infected cells were significantly lower than those in the uninfected cells and CRAd-GFP-infected cells.

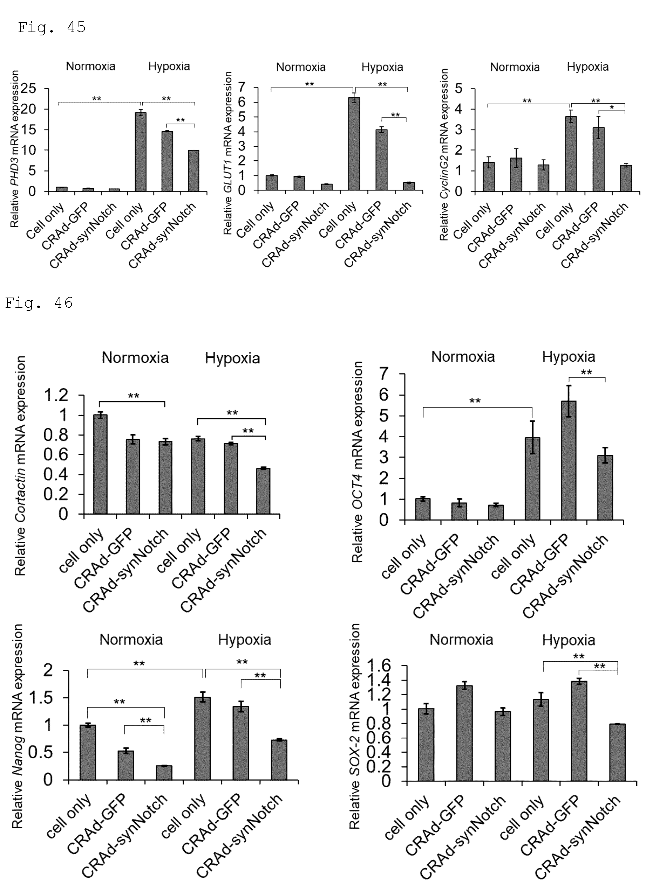

Fig. 46 shows the results of analyzing the expression levels of Cortactin (Fig. 46 (upper left)), OCT4 (Fig. 46 (upper right), Nanog (Fig. 46 (lower left)), and SOX-2 (Fig. 46 (lower right)), which are genes downstream of CD46, in the DU145 cells. As shown in Fig. 46, under hypoxic conditions, the expression levels of Cortactin, OCT4, Nanog, and SOX-2 in the CRAd-synNotch-infected cells were significantly lower than those in the uninfected cells and CRAd-GFP-infected cells.

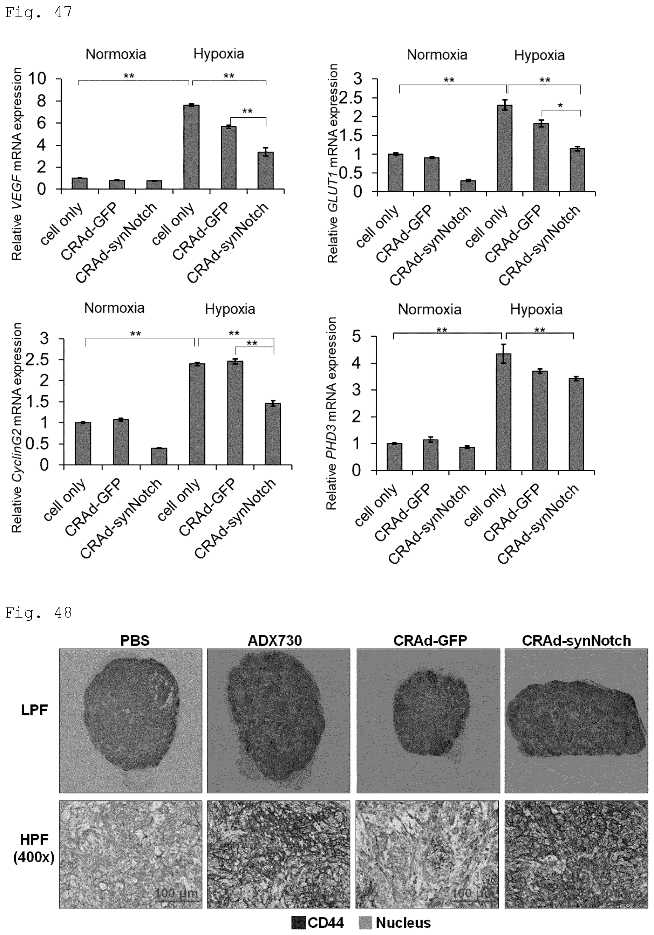

Fig. 47 shows the results of analyzing the expression levels of VEGF (Fig. 47 (upper left)), GLUT1 (Fig. 47 (upper right)), CyclinG2 (Fig. 47 (lower left)), and PHD3 (Fig. 47 (lower right)), which are genes downstream of HIF-1α, in the DU145 cells. As shown in Fig. 47, under hypoxic conditions, the expression levels of VEGF, GLUT1, CyclinG2, and PHD3 in the CRAd-synNotch-infected cells were significantly lower than those in the uninfected cells and CRAd-GFP-infected cells.

### 2.14 Immunohistochemical Staining of CD44 in T24 Mouse Model

As described in item 1.11, 1 × 10⁶ T24 cells were subcutaneously inoculated into nude mice to form a tumor. Thereafter, PBS or 1 × 10⁹ ifu of ADX730, CRAd-GFP, or CRAd-synNotch was intratumorally administered. The tumor was excised 24 hours after administration, and the expression of CD44 was investigated by immunohistochemical staining. Fig. 48 shows the results. As shown in the lower part of Fig. 48, a significant increase in CD44 expression was observed on the cell membrane of the tumor to which CRAd-synNotch or ADX730 was administered, compared with in the tumor to which CRAd-GFP or PBS was administered.

### 3. Discussion

The results described above show that CRAd-synNotch has an antitumor effect. The results also suggest that the antitumor effect may be attained through the suppression of expression of genes downstream of CD44 by the decoy function of the CD44 extracellular part contained in CRAd-synNotch and the suppression of expression of genes downstream of HIF-1α by the HIF-3α4 part contained in CRAd-synNotch. Furthermore, the results suggest that the HIF-3α4 part is cleaved from the fusion protein after expression.

## Claims

1. An oncolytic adenoviral vector comprising a nucleic acid, the nucleic acid comprising:
a base sequence (I) having a structure in which the following base sequences are linked in the order of (A)-(B)-(C):
(A) a base sequence encoding a protein having a CD44 extracellular function,
(B) a base sequence encoding a protein having a Notch core region function, and
(C) a base sequence encoding a protein having an HIF-3α4 function; and
a base sequence (II) comprising:
(D) a base sequence containing a COX-2 promoter;
(E) a base sequence encoding E1A protein placed under the control of the COX-2 promoter; and
(F) a base sequence encoding E1B protein placed under the control of the COX-2 promoter,
wherein the base sequence (I) and the base sequence (II) are arranged in the same orientation.

2. An oncolytic adenoviral vector comprising a nucleic acid, the nucleic acid comprising:
a base sequence (I) having a structure in which a base sequence (A), a base sequence (B), and a base sequence (C) are linked in the order of (A)-(B)-(C); and
a base sequence (II) comprising:
(D) a base sequence containing a COX-2 promoter;
(E) a base sequence encoding E1A protein placed under the control of the COX-2 promoter; and
(F) a base sequence encoding E1B protein placed under the control of the COX-2 promoter,
wherein the base sequence (I) and the base sequence (II) are arranged in the same orientation,
the base sequence (A) being:
(a-1): a base sequence consisting of the base sequence of SEQ ID NO: 1, or
(a-2): a base sequence consisting of a base sequence having a sequence identity of 85% or more to the base sequence of (a-1), and encoding a protein that can bind to hyaluronic acid,
the base sequence (B) being:
(b-1): a base sequence consisting of the base sequence of SEQ ID NO: 2, or
(b-2): a base sequence consisting of a base sequence having a sequence identity of 85% or more to the base sequence of (b-1), and encoding a protein that can be cleaved by a protease,
the base sequence (C) being:
(c-1): a base sequence consisting of the base sequence of SEQ ID NO: 3, or
(c-2): a base sequence consisting of a base sequence having a sequence identity of 85% or more to the base sequence of (c-1), and encoding a protein that can bind to HIF-1α,
the base sequence (D) being:
(d-1): a base sequence consisting of the base sequence of SEQ ID NO: 4, or
(d-2): a base sequence consisting of a base sequence having a sequence identity of 85% or more to the base sequence of (d-1), and encoding a promoter whose expression is induced under COX-2 expression-inducing conditions,
the base sequence (E) being:
(e-1): a base sequence consisting of the base sequence of SEQ ID NO: 5, or
(e-2): a base sequence consisting of a base sequence having a sequence identity of 85% or more to the base sequence of (e-1), and encoding a protein having E1A function, and
the base sequence (F) being:
(f-1): a base sequence consisting of the base sequence of SEQ ID NO: 6, or
(f-2): a base sequence consisting of a base sequence having a sequence identity of 85% or more to the base sequence of (f-1), and encoding a protein having E1B function.

3. The oncolytic adenoviral vector according to claim 1, wherein
the base sequence (A) is:
(a-1): a base sequence consisting of the base sequence of SEQ ID NO: 1, or
(a-2): a base sequence consisting of a base sequence having a sequence identity of 85% or more to the base sequence of (a-1), and encoding a protein that can bind to hyaluronic acid;
the base sequence (B) is:
(b-1): a base sequence consisting of the base sequence of SEQ ID NO: 2, or
(b-2): a nucleic acid consisting of a base sequence having a sequence identity of 85% or more to the base sequence of (b-1), and encoding a protein that can be cleaved by a protease in a protein encoded by the nucleic acid having a structure in which (A), (B), and (C) are linked in this order,
the base sequence (C) is:
(c-1): a nucleic acid consisting of the base sequence of SEQ ID NO: 3, or
(c-2): a nucleic acid consisting of a base sequence having a sequence identity of 85% or more to the base sequence of (c-1), and encoding a protein that can bind to HIF-1α,
the base sequence (D) is:
(d-1): a base sequence consisting of the base sequence of SEQ ID NO: 4, or
(d-2): a base sequence consisting of a base sequence having a sequence identity of 85% or more to the base sequence of (d-1), and encoding a promoter whose expression is induced under COX-2 expression-inducing conditions,
the base sequence (E) is:
(e-1): a base sequence consisting of the base sequence of SEQ ID NO: 5, or
(e-2): a base sequence consisting of a base sequence having a sequence identity of 85% or more to the base sequence of (e-1), and encoding a protein having E1A function, and
the base sequence (F) is:
(f-1): a base sequence consisting of the base sequence of SEQ ID NO: 6, or
(f-2): a base sequence consisting of a base sequence having a sequence identity of 85% or more to the base sequence of (f-1), and encoding a protein having E1B function.

4. An oncolytic adenoviral vector comprising a nucleic acid, the nucleic acid comprising:
a base sequence (III) being:
(g-1) a base sequence consisting of the base sequence of SEQ ID NO: 7,
(g-2) a base sequence consisting of a base sequence having a sequence identity of 85% or more to the base sequence of SEQ ID NO: 7, and having a structure in which a portion encoding a protein that can bind to hyaluronic acid, a portion encoding a protein that can be cleaved by a protease, and a portion encoding a protein that can bind to HIF-1α are linked in this order, or
(g-3) a base sequence consisting of a base sequence having a sequence identity of 85% or more to the base sequence of SEQ ID NO: 7, and encoding a protein having an anticancer effect; and
a base sequence (II) comprising:
(D) a base sequence containing a COX-2 promoter;
(E) a base sequence encoding E1A protein placed under the control of the COX-2 promoter; and
(F) a base sequence encoding E1B protein placed under the control of the COX-2 promoter,
wherein the base sequence (III) and the base sequence (II) are arranged in the same orientation,
the base sequence (D) being:
(d-1): a base sequence consisting of the base sequence of SEQ ID NO: 4, or
(d-2): a base sequence consisting of a base sequence having a sequence identity of 85% or more to the base sequence of (d-1), and encoding a promoter whose expression is induced under COX-2 expression-inducing conditions,
the base sequence (E) being:
(e-1): a base sequence consisting of the base sequence of SEQ ID NO: 5, or
(e-2): a base sequence consisting of a base sequence having a sequence identity of 85% or more to the base sequence of (e-1), and encoding a protein having E1A function, and
the base sequence (F) being:
(f-1): a base sequence consisting of the base sequence of SEQ ID NO: 6, or
(f-2): a base sequence consisting of a base sequence having a sequence identity of 85% or more to the base sequence of (f-1), and encoding a protein having E1B function.

5. The oncolytic adenoviral vector according to any one of claims 1 to 4, further comprising (H) a base sequence encoding an Ad5/35 chimeric fiber protein.

6. The oncolytic adenoviral vector according to claim 5, wherein the base sequence (H) is:
(h-1): a base sequence consisting of the base sequence of SEQ ID NO: 8, or
(h-2): a base sequence consisting of a base sequence having a sequence identity of 85% or more to the base sequence of (h-1), and encoding a protein that can bind to CD46.

7. The oncolytic adenoviral vector according to any one of claims 2 to 4 and 6, wherein the protease is an ADAM protease or γ-secretase.

8. An anticancer composition comprising the oncolytic adenoviral vector according to any one of claims 1 to 4 and 6.

9. The anticancer composition according to claim 8, which is an injection.

10. The anticancer composition according to claim 8 for use in the treatment of COX-2 positive cancer.

11. The anticancer composition according to claim 8 for use in the treatment of at least one selected from the group consisting of bladder cancer, breast cancer, and prostate cancer.
